# EUROPEAN PATENT SPECIFICATION

(11) **EP 2 742 351 B1**
(45) Date of publication and mention of the grant of the patent: **20.01.2016**
(21) Application number: 12756810.3
(22) Date of filing: 10.08.2012
(51) Int. Cl.: G01N 33/50, G01N 33/68

(54) **MATERIALS AND METHODS FOR DETERMINING SENSITIVITY POTENTIAL OF COMPOUNDS**
MATERIALIEN UND VERFAHREN ZUR BESTIMMUNG DES EMPFINDLICHKEITSPOTENZIALS VON VERBINDUNGEN
ÉCHANTILLONS ET PROCÉDÉS POUR DÉTERMINER LE POUVOIR DE SENSIBILISATION DE COMPOSÉS

(30) Priority: 10.08.2011 GB 201113814
(43) Date of publication of application: 18.06.2014
(73) Proprietor: Electrophoretics Limited, Cobham, Surrey KT11 3EP (GB)
(72) Inventor: BUDDE, Petra, D-60437 Frankfurt am Main (DE); ZUCHT, Hans-Dieter, D-30539 Hannover (DE); SELZER, Stefan, D-66679 Losheim am See (DE); KONCAREVIC, Sasa, D-97526 Sennfeld (DE); KUHN, Karsten, D-65719 Hofheim am Taunus (DE); PIKE, Ian, Tonbridge Kent TN104LB (GB); JUNG, Stephan, D-55122 Mainz (DE)
(74) Representative: Sainz Pastor, Noelia
(86) International application number: PCT/GB2012/051952
(87) International publication number: WO 2013/021210

(56) References cited:
- WO-A1-2009/148669
- US-A1- 2004 248 100
- CAROLINE BAUCH ET AL: "Intralaboratory validation of four in vitro assays for the prediction of the skin sensitizing potential of chemicals", TOXICOLOGY IN VITRO, vol. 25, no. 6, 7 June 2011 (2011-06-07), pages 1162-1168, XP055040296, ISSN: 0887-2333, DOI: 10.1016/j.tiv.2011.05.030
- NATSCH ANDREAS ET AL: "The intra- and inter-laboratory reproducibility and predictivity of the KeratinoSens assay to predict skin sensitizers in vitro: Results of a ring-study in five laboratories", TOXICOLOGY IN VITRO, vol. 25, no. 3, 1 April 2011 (2011-04-01), pages 733-744, XP055040297, ISSN: 0887-2333, DOI: 10.1016/j.tiv.2010.12.014
- ROGER EMTER ET AL: "Performance of a novel keratinocyte-based reporter cell line to screen skin sensitizers in vitro", TOXICOLOGY AND APPLIED PHARMACOLOGY, vol. 245, no. 3, 15 June 2010 (2010-06-15) , pages 281-290, XP055040275, ISSN: 0041-008X, DOI: 10.1016/j.taap.2010.03.009
- GRAHAM ELLIS ET AL: "Development of a high-throughput keratinocyte-based standard assay to detect skin sensitizers based on ARE-dependent gene activity", TOXICOLOGY LETTERS, vol. 189, 1 September 2009 (2009-09-01), page S69, XP055040276, ISSN: 0378-4274, DOI: 10.1016/j.toxlet.2009.06.207
- HELM ET AL: "Proteomic approach to characterize the effect of skin sensitizers on human primary keratinocytes", TOXICOLOGY LETTERS, ELSEVIER BIOMEDICAL PRESS, AMSTERDAM, NL, vol. 172, 4 September 2007 (2007-09-04), page S8, XP022229379, ISSN: 0378-4274
- N. LAMBRECHTS ET AL: "Assessment of Chemical Skin-Sensitizing Potency by an In Vitro Assay Based on Human Dendritic Cells", TOXICOLOGICAL SCIENCES, vol. 116, no. 1, 7 April 2010 (2010-04-07) , pages 122-129, XP055035599, ISSN: 1096-6080, DOI: 10.1093/toxsci/kfq108
- LAMBRECHTS N ET AL: "THP-1 monocytes but not macrophages as a potential alternative for CD34<+> dendritic cells to identify chemical skin sensitizers", TOXICOLOGY AND APPLIED PHARMACOLOGY, ACADEMIC PRESS, US, vol. 236, no. 2, 15 April 2009 (2009-04-15), pages 221-230, XP026029805, ISSN: 0041-008X, DOI: 10.1016/J.TAAP.2009.01.026 [retrieved on 2009-02-11]

## Description

### Field of the Invention

The present invention relates to in vitro proteomic analysis of cells to determine the sensitizing potential (including allergic potential) of compounds on said cells. Several protein markers have been identified which allow cellular based analysis to determine whether a compound has allergic or irritant potential. Particularly, but not exclusively, the invention provides assays for determining whether a test chemical has sensitizing potential of contact, i.e. on skin.

### Background of the Invention

Allergy is a type 1 hypersensitive disorder of the immune system. Common allergic reactions include asthma and contact dermatitis. Worldwide the occurrence of allergic diseases is steadily increasing. Allergic disorders have a negative impact on a patient's professional and social life. The costs to the healthcare systems of treating allergic diseases are substantial and increase with the corresponding rise in prevalence. Allergic contact dermatitis (ACD) is accepted to be the most prevalent form of immunotoxicity found in humans. ACD is a T cell mediated delayed skin hypersensitivity which develops after repeated exposure to common metals and a variety of different chemicals and cosmetics. Common chemical contact sensitizers are cinnamaldehyde (CA), dinitrochlorobenzene (DNCB), glyoxal, eugenol, p- phenylenediamine (PPD), and tetramethylthiuram (TMTD). PPD is a chemical substance that is widely used as a permanent hair dye, in textiles, temporary tattoos, photographic developer, printing inks, black rubber, oils, greases and gasoline.

Many occupational allergens causing allergic contact dermatitis are chemicals (or haptens) that have to bind to a carrier protein to trigger a delayed immune response.

Currently, the sensitizing potential of a chemical is assessed in animal experiments such as the guinea pig maximization test (Magnussen and Kligman, 1969) and the local lymph node assay (LLNA) (Kimber et al. 1995). However, the European Directive 86/609/EEC and the 7th Amendment to the Cosmetics Directive enforce an animal testing ban for all cosmetic ingredients since March 2009. Moreover, a marketing ban is in force for cosmetic products containing ingredients tested in animals for all endpoints except repeated dose toxicity, for which the deadline is 2013.

Much research has been devoted to the development of *in vitro* and *in silico* predictive testing methods. However, validated *in vitro* assays for identification and screening of contact sensitizing chemicals are not available.

Chemical allergens are typically small with masses under 1000 daltons, are electrophilic or hydrophilic and can react with nucleophilic amino acids of proteins. Such reactive low molecular weight chemicals can become allergenic when they bind to larger carrier proteins in the body to form hapten- protein conjugates. Some chemical allergens are not inherently allergenic and must undergo metabolic transformation (pro-hapten) or oxidation (pre-hapten) before participating in an allergic response. For example eugenol is considered a pro-hapten, whereas isoeugenol and PPD are classified as pre-haptens.

The skin is the largest organ of the human body and represents a large contact site for potential allergy inducing chemicals. It consists of Langerhans cells (LCs, antigen-presenting dendritic cells), T-lymphocytes, natural killer cells and keratinocytes actively participating in an allergic response. About 95% of all epidermal cells are keratinocytes and are the first cells to encounter foreign antigens. During the induction phase of ACD a chemical allergen binds to an epidermal protein to form a hapten-carrier protein complex. As keratinocytes also express drug metabolizing enzymes they also participate in the conversion of pro-drugs to sensitizers. Upon contact with a chemical hapten keratinocytes produce a number of cytokines such as interleukin-18 (IL-18) and tumour necrosis factor alpha (TNF-alpha) inducing migration of LCs to local lymph nodes. Hapten-protein conjugates are recognized by dendritic cells (DCs) which internalize process and transport antigen to the lymph node and present it to T-lymphocytes.

After uptake and processing of foreign or self antigens in peripheral tissues, LCs undergo a complex maturation process. Therefore, such test systems comprising primary keratinocytes, human immortalised keratinocytes (HaCaT), human keratinocyte cell lines such as NCTC 2544, epithelial cells or human reconstructed epidermal models such as EpiDerm (MatTek Corporation USA) or EpiSkin ((SkinEthic, France) could be useful to develop alternative approaches for predicting the sensitizing potential of chemicals.

The cellular response to irritants and allergens is manifested in two principal ways. Initial exposure is likely to trigger altered gene expression which is subsequently followed by changes in the protein composition of the exposed cells. It is to be appreciated that potential markers of irritant or allergic exposure may be found through the analysis of gene expression or by proteomic analysis of model systems. It is the primary objective of the present invention to provide protein markers whose expression is known to increase or decrease in cells exposed to different classes of chemical compounds. The skilled person would understand that changes in protein levels may also be accompanied, and are often preceded by a parallel change in gene expression and such gene expression changes are within the scope of the present invention.

Whilst there have been very few studies on protein expression changes in model systems for chemical safety testing, there have been several studies on the effects of irritants and allergens on gene expression profiles:
Previous studies have focused on developing *in vitro* sensitization assays based on measuring cytokine mRNA expression and production in murine and human keratinocyte cell lines (Corsini et al. 2009; Van Och et al. 2005). For example the human keratinocyte cell line NCTC2544 responds to application of contact sensitizers with production of interleukin-18 (IL-18). In contrast, irritant and respiratory sensitizers failed to increase IL-18 production (Corsini et al.2009).

The study of Helm S. et al. (2007) aimed to characterize the molecular effects of skin sensitizer on primary human keratinocytes as first events in contact hypersensitivity (CHS) to Ni²⁺ ions.

Others have focused on measuring the activation of a cellular toxicity pathway that recognizes various electrophilic chemicals. This pathway comprises the cellular sensor protein Kelch-like ECH-associated protein (Keapl) and the transcription factor Nrf2, which binds to the antioxidant response element (ARE) in the promoter of phase 2 detoxification genes(Natsch and Emter 2008; Natsch 2010). Activation of the Keap2/Nrf2 pathway has been utilized by Givaudan Fragance Research (Duebendorf, Switzerland) to develop the human KeratinoSens assay which is based on expressing the luciferase gene under the control of the ARE of the AKR1C2 gene in HaCaT keratinocytes (Emter et al. (2010); Ellis G. et al. (2009)). After incubation with a test chemical induction of luciferase activation and cytotoxicity are measured to identify skin sensitizers. The KeratinoSens assay was used to screen 43 sensitizers, and 24 non-sensitizers. The system correctly identified 38 sensitizer and 19 non-sensitizer.
Among the false negative sensitizer was phenyl benzoate which reacts with Lysine residue a reaction which is not identified by ARE-based assays (Natsch et al. 2010; Emter et al. 2010). This means that additional biomarker from other pathways are needed to identify a full spectrum of chemical allergens.

Yoshikawa et al. (2010) described the use of two strains of human keratinocytes, bulge-derived keratinocytes (BDKs), human neonatal epidermal keratinocytes (NHEKs), and the human monocytic leukemia cell line THP-1 to identify genes responding to incubation with chemical sensitizers. BDKs, NEHKs and THP-1 showed high reactivity to the chemical sensitizer DNCB with upregulation of the Nrf2 pathway.
However, great differences in the gene expression of potential biomarkers including interleukin 1B (IL1B), interleukin-8 (IL8) or inhibitor of DNA binding 2 (ID2) was observed between the three cell types. For example gene expression of IL1B and IL-8 was much higher induced by DNCB in BDKs compared to THP-1 cells. This means that different molecular pathways are induced in different cell types and different sets of biomarkers are needed in different cellular test systems.

Vandenbriel et al. (2010) analyzed changes in gene expressing in HaCaT cells using Affymetrix U133 Plus 2.0 Arrays exposed to eight known sensitizers and six irritants. Based on support vector machine (SVM) algorithms 13 most discriminating genes between sensitizer and irritants were selected and included genes from oxidative stress pathways and Keap1-dependent genes.

Bauch C. et al. (2011) presents the validation of four assays addressing three different events during induction of skin sensitization with 23 different substances. These assays are (1) the Direct Peptide Reactivity Assay (DPRA) which uses synthetic peptides and HPLC analysis; (2)(3) dendritic cell activation assay based on the dendritic cell lines such as U-937 and TPH-1 and flow cytometric detection of the maturation markers CD54 and/or CD86 and (4) antioxidant response element (ARE)-dependent gene activity in a HaCaT reporter gene cell line.

Lambrechts N. et al. (2009) investigated whether human in vitro cultured THP-1 monocytes or macrophages displayed a similar expression profile for 13 predictive gene markers previously identified in human CD34⁺ progenitor-derived dendritic cells, an in vitro classification model for the prediction of chemical induced sensitization based on gene expression signature, which has the drawback of being exclusively derivable by cord blood. In a later study (Lambrechts N. et al. (2010)), the CD34-DC based assay was used for establishing the relative potential (from weak to extreme) of sensitizing compounds.

US2004248100 describes a method for identifying additional compounds to Saframycin A which bind to GAPDH.

WO2009/148669 describes methods for predicting the in vivo skin sensitizing activity of chemical compounds using a combination of mammalian cell models without the use of animals by measuring marker genes such as IL-8, TXN and TXN reductase.

Accordingly, there is still no consensus as to the most appropriate cell lines or genes to use as surrogate screens for chemical safety *in vitro.*

### Summary of the Invention

Starting from the assumption that allergic responses will be mediated by changes in protein expression, the present inventors have carried out a detailed proteomic analysis of keratinocytes exposed to known irritants and sensitizers to reveal putative markers. Surprisingly, there was virtually no overlap between previously reported gene regulations and proteins seen to change in response to exposure with different classes of chemicals. As a result the inventors have defined a small panel of 102 proteins that can serve as objective measures of allergic and skin irritation response in *in vitro* screens of chemical safety.

The invention allows methods to be carried out for predicting the sensitizing potential of chemical sensitizers using *in vitro* methods capable of replacing whole organism testing, based on the measurement of any one or more of these protein markers.

The sensitising potential of a compound includes its ability to cause an allergic reaction, or its ability to act as a non-allergenic irritant.

In a first aspect, there is provided an *in vitro* method for determining the sensitizing potential of a test compound, comprising the steps of
(a) contacting said test compound with a cell;
(b) determining the presence or a change in the level of expression of glyceraldehyde-3-phosphate dehydrogenase (GAPDH) and further marker proteins
   and
(c) determining the sensitizing potential of said test compound based on said presence or change in level of expression wherein a change in the presence or level of expression of said one or more marker proteins is indicative of said test compound having sensitizing potential,
wherein said further marker proteins are selected from Table 1, Table 1 (A) Group 1; Table 1 (B) Group 2; or Table 1 (C) Group 3 or a combination thereof, and wherein the cell is representative of a mammalian skin cell.

Table 1 contains 102 protein markers. In preferred embodiments of the invention, the method determines the presence or a change in level of expression of a plurality of these protein markers. Thus, the method according to the first aspect of the invention may determine the presence or change in level of expression of 2, 3, 5, 10, 20, 30, 40, 50, 60, 70, 80, 90, 100, or more protein markers provided in Table 1.

Alternatively, the method may comprises determining the presence or change in level of expression of at least 5%, 10%, 20%, 30%, 40%, 50%, 60%, 70%, 80%, 90% of the protein markers provided in Table 1.

The method according to this and other aspects of the invention may comprise comparing said presence of level of expression of the one or more protein markers with a reference level. In light of the present disclosure, the skilled person is readily able to determine a suitable reference level, e.g. by deriving a mean and range of values from cells derived from the same, or equivalent cell line. In certain embodiments, the method of this and other aspects of the invention may further comprise determining a reference level for one or more of said marker proteins, above which or below which the presence or amount of said one or more protein markers being expressed in the cell in contact with the test compound can be considered to indicate the sensitizing potential of the compound.

However, the reference level is preferably a pre-determined level, which may for example be provided in the form of an accessible data record. The test compound may be contacted with any cell. Preferably, the mammalian skin cell is selected from the group consisting of a primary keratinocyte,a keratinocyte derived cell line, an epidermal keratinocytes, bulge-derived keratinocytes, foreskin keratinocytes, a human cell with keratinocytic properties, a cell derived from HaCaT cell line and a cell derived from NCTC2544 cell line.

The method according to this aspect of the invention may be used to determine the contact sensitizing potential of the test compound by determining the presence or a change in expression level of further marker proteins provided in Table 1 (A) group 1 when the test compound is contacted with the cell. The further marker proteins may be 2, 4, 5, 6, 8 or more selected from Table 1 (A) group 1; or may include all 12 marker proteins.

In a further embodiment, the method may be used to determine the irritating potential of a test compound by determining the presence or a change in expression level of further marker proteins provided in Table 1 (B) Group 2 when the test compound is contacted with the cell. The further marker proteins may be 2, 3 or more selected from Table 1 (B) Group 2, or may include all 6 marker proteins.

**Table 1: Protein markers of chemical sensitizing effect**

| **(A) Group 1 -** top 12 skin sensitizer markers; **(B) Group 2 -** top 6 markers for skin irritation (*); **(C) Group 3 -** additional general markers of sensitizing and irritation effect | | | | | |
|---|---|---|---|---|---|
| **SEQ ID No.** | **Protein Name** | **IPI Accession Number** | **Gene name** | **UniProt Accession** | **Change of cell lyzate protein level on exposure to known sensitizers or irritant(*)** |
| | **A Group 1** | | | | |
| 1 | Glyceraldehyde-3-phosphate dehydrogenase | IPI00219018 | GAPDH | P04406 | Increased |
| 2 | Thioredoxin | IPI00216298 | TXN | P10599 | Increased |
| 3 | Isoform Beta of LIM domain and actin-binding protein 1 | IPI00008918 | LIMA1 | Q9UHB6 | Increased |
| 4 | Isoform 1 of Metallothionein-1G | IPI00008752 | MT1G | P13640 | Increased |
| 5 | 60S ribosomal protein L31 | IPI00026302 | RPL31 | P62899 | Increased |
| 6 | Annexin A3 | IPI00024095 | ANXA3 | P12429 | Increased |
| 7 | Eukaryotic translation initiation factor 3 subunit C | IPI00016910 | EIF3CL;EI F3C | Q99613 | Increased |
| 8 | Rho GDP-dissociation inhibitor 1 | IPI00003815 | ARHGDIA | P52565 | Decreased |
| 9 | Poly(rC)-binding protein 1 | IPI00016610 | PCBP1 | Q15365 | Increased |
| 10 | Nucleosome assembly protein 1-like 1 | IPI00023860 | NAP1L1 | P55209 | Increased |
| 11 | Heat shock 70 kDa protein 1 | IPI00304925 | HSPA1H; HS PA1A | P08107 | Increased |
| 12 | Aldo-keto reductase family 1 member C2 | IPI00005668 | AKR1C2 | P52895 | Increased |

| | **B Group 2** | | | | |
|---|---|---|---|---|---|
| 13 | Ribosomal protein L14 variant | IPI00555744 | RPL14 | P50914 | Increased* |
| 14 | Epidermal growth factor receptor kinase substrate 8 | IPI00290337 | EPS8 | Q12929 | Increased* |
| 15 | Plasminogen activator inhibitor 2 | IPI00007117 | SERPINB2 | P05120 | Increased* |
| 16 | Isoform 1 of Voltage-dependent anion-selective channel protein 3 | IPI00031804 | VDAC3 | Q9Y277 | Increased* |
| 17 | Annexin A1 | IPI00218918 | ANXA1 | P04083 | Increased* |
| 18 | Cystatin-B | IPI00021828 | CSTB | P04080 | Increased* |

| | **C Group 3** | | | | |
|---|---|---|---|---|---|
| 19 | Isoform 1 of Clathrin heavy chain 1 | IPI00024067 | CLTC | Q00610 | Decreased |
| 20 | Elongation factor 1-alpha 1 | IPI00396485 | EEF1A1 | P68104 | Decreased |
| 21 | Annexin A2 isoform 1 | IPI00418169 | ANXA2 | P07355 | Increased |
| 22 | cDNA FLJ54535, highly similar to Pyruvate kinase isozymes | IPI00910979 | PKM2 | B4DUU6 | Increased |
| 23 | Isoform alpha-enolase of Alpha-enolase | IPI00465248 | ENO1 | P06733 | Increased |
| 24 | Neuroblast differentiation-associated protein AHNAK | IPI00021812 | AHNAK | Q09666 | Increased |
| 25 | Isoform 1 of Heat shock cognate 71 kDa protein | IPI00003865 | HSPA8 | P11142 | Decreased |
| 26 | Keratin, type I cytoskeletal 14 | IPI00384444 | KRT14 | P02533 | Decreased |
| 27 | Ubiquitin and ribosomal protein S27a precursor | IPI00179330 | UBC;UBB;R PS27A | P62979 | Decreased |
| 28 | FLJ54957, highly similar to Transketolase | IPI00643920 | TKT | B4DE31 | Increased |
| 29 | Galectin-1 | IPI00219219 | LGALS1 | P09382 | Increased |
| 30 | Isoform M1 of Pyruvate kinase isozymes M1/M2 | IPI00220644 | PKM2 | E7EUQ8 | Decreased |
| 31 | Tubulin beta chain | IPI00011654 | TUBB | Q6P602 | Decreased |
| 32 | Isoform 2 of Nucleophosmin | IPI00220740 | NPM1 | P06748 | Decreased |
| 33 | Isoform 1 of Nucleoside diphosphate kinase B | IPI00026260 | NME1;NME2 | P22392 | Increased |
| 34 | Thrombospondin-1 | IPI00296099 | THBS1 | P07996 | Decreased |
| 35 | cDNA FLJ55792, highly similar to L-lactate dehydrogenase A chain | IPI00217966 | LDHA | P00338 | Increased |
| 36 | Keratin, type II cytoskeletal 5 | IPI00009867 | KRT5 | P13647 | Increased |
| 37 | HSPA5 protein | IPI00003362 | HSPA5 | P11021 | Increased |
| 38 | Thymosin beta-10 | IPI00220827 | TMSB10 | P63313 | Decreased |
| 39 | Isoform Long of Laminin subunit gamma-2 | IPI00015117 | LAMC2 | Q2M1N2 | Increased |
| 40 | Keratin, type II cytoskeletal 1 | IPI00220327 | KRT1 | P04264 | Decreased |
| 41 | ADP/ATP translocase 2 | IPI00007188 | SLC25A5 | P05141 | Decreased |
| 42 | Actin, cytoplasmic 1 | IPI00021439 | ACTB | P60709 | Increased |
| 43 | Epiplakin 1 | IPI00010951 | EPPK1 | P58107 | Decreased |
| 44 | Cofilin-1 | IPI00012011 | CFL1 | P23528 | Increased |
| 45 | Laminin subunit beta-3 | IPI00299404 | LAMB3 | Q13751 | Increased |
| 46 | Isoform 1 of Heterogeneous nuclear ribonucleoprotein Q | IPI00018140 | SYNCRIP | 060506 | Decreased |
| 47 | Hemoglobin subunit gamma-1 | IPI00220706 | HBG1 | E7CYP2 | Increased |
| 48 | Glutathione S-transferase P | IPI00219757 | GSTP1 | P09211 | Increased |
| 49 | Fructose-bisphosphate aldolase A | IPI00465439 | ALDOA | P04075 | Increased |
| 50 | 60S ribosomal protein L18 | IPI00215719 | RPL18 | Q07020 | Decreased |
| 51 | EEF2 Elongation factor 2 | IPI00186290 | EEF2 | P13639 | Decreased |
| 52 | RPL4 11 kDa protein | IPI00792159 | RPL4 | P36578 | Increased |
| 53 | RPL6 60S ribosomal protein L6 | IPI00329389 | RPL6 | Q02878 | Increased |
| 54 | Keratin 7 | IPI00847342 | KRT7 | P08729 | Increased |
| 55 | Putative uncharacterized protein HNRNPA2B1 | IPI00386854 | HNRNPA2B1 | P22626 | Increased |
| 56 | Protein S100-A6 | IPI00027463 | S100A6 | P06703 | Increased |
| 57 | Tudor domain-containing protein 6 | IPI00030153 | TDRD6 | 060522 | Increased |
| 58 | Coiled-coil domain-containing protein KIAA1407 | IPI00477003 | KIAA1407 | Q8NCU4 | Decreased |
| 59 | Histone H2B type 1-L | IPI00018534 | HIST1H2BL | Q99880 | Increased |
| 60 | Peptidyl-prolyl cis-trans isomerase A | IPI00419585 | PPIA | A8K220 | Increased |
| 61 | cDNA FLJ53068, highly similar to Adenylyl cyclase-associated protein 1 | IPI00643567 | CAP1 | Q01518 | Decreased |
| 62 | Tu translation elongation factor, mitochondrial precursor | IPI00027107 | TUFM | P49411 | Decreased |
| 63 | SH3 domain binding glutamic acid-rich protein like 3 | IPI00010402 | SH3BGRL3 | Q86Z22 | Increased |
| 64 | Cornifin-B | IPI00304903 | SPRR1B | P22528 | Decreased |
| 65 | Isoform Beta-4C of Integrin beta-4 | IPI00027422 | ITGB4 | P16144 | Decreased |
| 66 | 60S ribosomal protein L35 | IPI00412607 | RPL35 | P42766 | Increased |
| 67 | 60S ribosomal protein L34 | IPI00219160 | RPL34 | P49207 | Decreased |
| 68 | Protein S100-A8 | IPI00007047 | S100A8 | P05109 | Decreased |
| 69 | HSP90B1 Endoplasmin | IPI00027230 | HSP90B1 | P14625 | Increased |
| 70 | Hemoglobin subunit alpha | IPI00410714 | HBA2;HBA1 | P69905 | Increased |
| 71 | SFN Isoform 1 of 14-3-3 protein sigma | IPI00013890 | SFN | P31947 | Increased |
| 72 | HSP90AA1 heat shock 90kDa protein 1, alpha isoform 1 | IPI00382470 | HSP90AA1 | P07900 | Increased |
| 73 | S100A9 Protein S100-A9 | IPI00027462 | S100A9 | P06702 | Decreased |
| 74 | cDNA FLJ54408, highly similar to Heat shock 70 kDa protein 1 | IPI00911039 | HSP70.1 | B4E3B6 | Increased |
| 75 | Keratin, type I cytoskeletal 17 | IPI00450768 | KRT17 | Q04695 | Decreased |
| 76 | Heat shock protein beta-1 | IPI00025512 | HSPB1 | P04792 | Decreased |
| 77 | FASN Fatty acid synthase | IPI00026781 | FASN | P49327 | Decreased |
| 78 | KRT6A Keratin, type II cytoskeletal 6A | IPI00300725 | KRT6A | P02538 | Decreased |
| 79 | DSP Isoform DPI of Desmoplakin | IPI00013933 | DSP | P15924 | Decreased |
| 80 | PRDX1 Peroxiredoxin-1 | IPI00000874 | PRDX1 | Q06830 | Increased |
| 81 | Protein disulfide-isomerase | IPI00010796 | P4HB | P07237 | Increased |
| 82 | Histone H1.4 | IPI00217467 | HIST1H1E | P10412 | Decreased |
| 83 | Isoform 1 of 60S ribosomal protein L12 | IPI00024933 | RPL12 | P30050 | Decreased |
| 84 | Tubulin alpha-4A chain | IPI00007750 | TUBA4A | P68366 | Decreased |
| 85 | Tubulin beta-3 chain | IPI00013683 | TUBB3 | Q13509 | Decreased |
| 86 | 14-3-3 protein gamma | IPI00220642 | YWHAG | P61981 | Increased |
| 87 | Vimentin | IPI00418471 | VIM | P08670 | Increased |
| 88 | Filamin-A | IPI00302592 | FLNA | P21333 | Increased |
| 89 | Annexin A5 | IPI00329801 | ANXA5 | P08758 | Increased |
| 90 | 40S ribosomal protein S28 | IPI00719622 | RPS28 | P62857 | Decreased |
| 91 | 60S acidic ribosomal protein P0 | IPI00008530 | RPLP0 | P05388 | Increased |
| 92 | Laminin alpha-3 chain variant 1 | IPI00377045 | LAMA3 | B0YJ32 | Increased |
| 93 | Histone H2A type 1-H | IPI00081836 | HIST1H2AH | Q96KK5 | Decreased |
| 94 | Eukaryotic initiation factor 4A-I | IPI00025491 | EIF4A1 | P60842 | Increased |
| 95 | Moesin | IPI00219365 | MSN | P26038 | Increased |
| 96 | Keratin, type I cytoskeletal 16 | IPI00217963 | KRT16 | P08779 | Decreased |
| 97 | Phosphoglycerate mutase | IPI00453476 | PGAM1 | P18669 | Increased |
| 98 | Protein disulfide-isomerase A3 | IPI00025252 | PDIA3 | P30101 | Increased |
| 99 | Peptidyl-prolyl cis-trans isomerase B | IPI00646304 | PPIB | P23284 | Increased |
| 100 | Galectin-7 | IPI00219221 | LGALS7;LG ALS7B | P47929 | Decreased |
| 101 | cDNA FLJ45706 fis, clone FEBRA2028457, highly similar to Nucleolin | IPI00444262 | NCL | E7EX81 | Decreased |
| 102 | Peroxiredoxin-2 | IPI00027350 | PRDX2 | P32119 | Increased |

The determination may involve direct quantification of nucleic acid or protein levels, or it may involve indirect quantification, e.g. using an assay that provides a measure that is correlated with the amount of marker protein present. Accordingly, determining the presence or level of expression of the one or more marker proteins may comprise
(a) contacting the cell with at least one specific binding member that selectively binds to said marker protein or nucleic acid sequence encoding said marker protein ; and
(b) detecting and/or quantifying a complex formed by said specific binding member and the marker protein or nucleic acid sequence encoding said marker protein.

The specific binding member may be an antibody or antibody fragment that specifically and selectively binds a marker protein. The determination may include preparing a standard curve using standards of known expression levels of the GADPH and the further marker proteins and comparing the reading obtained with the cell contacted with the test compound so as to derive a measure of the change in level of expression of the GADPH and the further marker proteins.

A variety of methods may be suitable for determining the presence or changes in level of expression of the one or more marker proteins: by way of a non-limiting example, these include Western blot, ELISA (Enzyme-Linked Immunosorbent Assay), RIA (Radioimmunoassay), Competitive EIA (Competitive Enzyme Immunoassay), DAS-ELISA (Double Antibody Sandwich-ELISA), Liquid Immunoarray technology), immunocytochemical or immunohistochemical techniques, techniques based on the use of protein microarrays that include specific antibodies, "dipstick" assays, affinity chromatography techniques and liquid binding assays. The specific binding member may be an antibody or antibody fragment that selectively binds the protein marker or part thereof. Any suitable antibody format may be employed.

A further class of specific binding members contemplated herein in accordance with any aspect of the invention comprise aptamers (including nucleic acid aptamers and peptide aptamers). Advantageously, an aptamer directed to a protein marker may be provided by a technique known as SELEX (Systematic Evolution of Ligands by Exponential Enrichment), described in US Patent Nos. 5,475,096 and 5,270,163.

In some embodiments of this and other aspects of the invention, the determination of the presence or the level of expression of one or more of the marker proteins may be performed by mass spectrometry. Techniques suitable for measuring the level of a protein marker selected from Table 1 are readily available to the skilled person and include techniques related to Selected Reaction Monitoring (SRM) and Multiple Reaction Monitoring (MRM) isotope dilution mass spectrometry including SILAC, AQUA (as disclosed in WO 03/016861, the entire content of which is specifically incorporated herein by reference) and TMT calibrator (as disclosed in WO 2008/110581; the entire content of which is specifically incorporated herein by reference).

By way of a reference level, a known or predicted protein marker derived peptide may be created by trypsin, ArgC, AspN or Lys-C digestion of said protein marker. In some cases, when employing mass spectrometry based determination of protein markers, the methods of the invention comprises providing a calibration sample comprising at least two different aliquots comprising the protein marker and/or at least one protein marker derived peptide, each aliquot being of known quantity and wherein said biological sample and each of said aliquots are differentially labelled with one or more isobaric mass labels. Preferably, the isobaric mass labels each comprise a different mass spectrometrically distinct mass marker group.

Accordingly, in a preferred embodiment of the invention, the method comprises determining the presence or expression level of GAPDH and further marker proteins selected from Table 1 in a cell contacted with a test compound by Selected Reaction Monitoring using one or more determined transitions for known protein marker derived peptides; comparing the peptide levels in the cell under test with peptide levels previously determined to represent contact sensitivity by the cell; and determining the sensitivity potential of the test compound based on changes in expression of GAPDH and said further marker proteins; wherein an increase in expression of GAPDH is indicative of said test compound having sensitizing potential.. The comparison step may include determining the amount of marker protein derived peptides from the treated cell with known amounts of corresponding synthetic peptides.
The synthetic peptides are identical in sequence to the peptides obtained from the cell, but may be distinguished by a label such as a tag of a different mass or a heavy isotope.

In some embodiments the marker derived peptides are selected from Table 5. In these embodiments the method may comprise determining the presence or expression level of further marker proteins in a cell contacted with a test compound by Selected Reaction Monitoring using one or more (or a plurality, e.g. two, three, four, five, eight, ten, fifteen, twenty, thirty, forty or all) determined transitions in Table 5; comparing the peptide levels in the cell under test with peptide levels previously determined to represent contact sensitivity by the cell; and determining the sensitivity potential of the test compound based on changes in expression of said one or more marker proteins. The comparison step may include determining the amount of marker protein derived peptides from the treated cell with known amounts of corresponding synthetic peptides. The synthetic peptides are identical in sequence to the peptides obtained from the cell, but may be distinguished by a label such as a tag of a different mass or a heavy isotope.

In a preferred embodiment the one or more determined transitions include one or more (or a plurality, e.g. two, three, four, five, eight, ten, twelve, or all fifteen) of:

| Analyte | Peptide Sequence | TMT label | m/z (Q1) | m/z (Q3) | fragme nt ion type | Colli sion energy | start time | stop time |
|---|---|---|---|---|---|---|---|---|
| HSPA8 | GTLDPVEK | light | 653.8 80455 | 611.33 588 | b4 | 29 | 9.5 | 11.6 |
| HSPA8 | GTLDPVEK | light | 653.8 80455 | 696.42 503 | y4 | 30 | 9.5 | 11.6 |
| HSPA8 | GTLDPVEK | light | 653.8 80455 | 811.45 1973 | y5 | 31 | 9.5 | 11.6 |
| HSPA8 | TVTNAVVTVPA YFNDSQR | light | 736.0 53596 | 640.36 2429 | b4 | 47 | 11.2 | 12.9 |
| HSPA8 | TVTNAVVTVPA YFNDSQR | Light | 736.0 53596 | 711.39 9543 | b5 | 36 | 11.2 | 12.9 |
| HSPA8 | TVTNAVVTVPA YFNDSQR | light | 736.0 53596 | 810.46 7957 | b6 | 32 | 11.2 | 12. 9 |
| S100A 8 | ALNSIIDVYHK | light | 574.3 35375 | 610.35 1865 | b4 | 31 | 11.4 | 12.9 |
| S100A 8 | ALNSIIDVYHK | light | 574.3 35375 | 723.43 5929 | b5 | 27 | 11.4 | 12.9 |
| S100A 9 | LGHPDTLNQGE FK | light | 635.3 4471 | 704.39 373 | y4 | 25 | 10 | 11.6 |
| S100A 9 | NIETIINTFHQ YSVK | light | 752.4 16646 | 682.37 2994 | b4 | 42 | 12.4 | 14.2 |
| S100A 9 | NIETIINTFHQ YSVK | light | 752.4 16646 | 720.42 503 | y4 | 35 | 12.4 | 14.2 |
| S100A 9 | NIETIINTFHQ YSVK | light | 752.4 16646 | 795.45 7058 | b5 | 38 | 12.4 | 14.2 |
| TYB10 | ETIEQEK | light | 662.8 67545 | 628.36 2429 | y3 | 34 | 8.4 | 10.5 |
| TYB10 | ETIEQEK | light | 662.8 67545 | 697.37 266 | b4 | 37 | 8.4 | 10.5 |
| TYB10 | ETIEQEK | light | 662.8 67545 | 757.40 5023 | y4 | 34 | 8.4 | 10.5 |

Other suitable methods for determining levels of protein expression include surface-enhanced laser desorption ionization-time of flight (SELDI-TOF) mass spectrometry; matrix assisted laser desorption ionization-time of flight (MALDI-TOF) mass spectrometry; electrospray ionization (ESI) mass spectrometry; as well as the preferred SRM.

In some embodiments, the determination of the presence or amount of the one or more protein markers comprises measuring the presence or amount of mRNA derived from the cell under test. The presence or level of mRNA encoding the protein marker in the cells contacted with the test compound provides a determination of whether the test compound has a sensitizing potential. Techniques suitable for measuring the level of protein marker encoding mRNA are readily available to the skilled person and include "real-time" reverse transcriptase PCR or Northern blots. The method of measuring the level of a protein marker encoding mRNA may comprise using at least one primer or probe that is directed to the sequence of the protein marker encoding gene or complement thereof. The at least one primer or probe may comprise a nucleotide sequence of at least 10, 15, 20, 25, 30 or 50 contiguous nucleotides that has at least 70%, 80%, 90%, 95%, 98%, 99% or 100% identity to a nucleotide sequence encoding the protein marker provided in Table 1 and Figure 10.

Preferably, the at least one probe or primer hybridises under stringent conditions to a protein marker encoding nucleic acid sequence.

The method of the invention may comprises contacting the cell with a binding member as described above, but also includes contacting the binding member with culture medium around the cells which may contain products secreted by the cells.

Further, it may be preferably to lyse the cell prior to contact with the binding member to increase contact directly or indirectly with the one or more marker proteins.

The binding members may be immobilised on a solid support. This may be in the form of an antibody array or a nucleic acid microarray. Arrays such as these are well known in the art. The solid support may be contacted with the cell lysate or culture medium surrounding the cell, thereby allowing the binding members to bind to the cell products or secreted products representing the presence or amount of the one or more marker proteins.

In some embodiments, the binding member is an antibody or fragment thereof which is capable of binding to a marker protein or part thereof. In other embodiments, the binding member may be a nucleic acid molecule capable of binding (i.e. complementary to) the sequence of the nucleic acid to be detected.

The method may further comprise contacting the solid support with a developing agent that is capable of binding to the occupied binding sites, unoccupied binding sites or the one or more marker proteins, antibody or nucleic acid.

The developing agent may comprise a label and the method may comprise detecting the label to obtain a value representative of the presence or amount of the one or more marker proteins, antibody or nucleic acid in the cell, cell culture medium or cell lysate.

The label may be, for example, a radioactive label, a fluorophor, a phosphor, a laser dye, a chromogenic dye, a macromolecular colloidal particle, a latex bead which is coloured, magnetic or paramagnetic, an enzyme which catalyses a reaction producing a detectable result or the label is a tag.

The method may comprise determining the presence or level of expression of GAPDH or further marker proteins or nucleic acids encoding GAPDH or further marker proteins in a single sample. For example, a plurality of binding members selected from Table 1, Table 1 (A) Group 1; Table 1 (B) Group 2; or Table 1 (C) Group 3 or a combination thereof, may be immobilised at predefined locations on the solid support. The number of binding members selected from Table 1 on the solid support may make up 10%, 20%, 30%, 40%, 50%, 60%, 70%, 80%, 90% or 100% of the total number of binding members on the support.

Alternatively, a plurality of mass features are selected for mass spectrometry techniques described above.

The binding member may be an antibody specific for a marker protein or a part thereof, or it may be a nucleic acid molecule which binds to a nucleic acid molecule representing the presence, increase or decrease of expression of a marker protein, e.g. an mRNA sequence.

The antibodies raised against specific marker proteins may be anti- to any biologically relevant state of the marker protein. Thus, for example, they can be raised against the unglycosylated form of a protein which exists in the body in a glycosylated form, against a precursor form of the protein, or a more mature form of the precursor protein, e.g. minus its signal sequence, or against a peptide carrying a relevant epitope of the marker protein.

In a second aspect of the invention, there is provided a kit for use in determining the sensitizing potential of a test compound *in vitro.*

The kit may be used in an *in vitro* method of determining sensitizing potential of a test compound. This method may be performed as part of a general screening of multiple samples, or may be performed on a single sample obtained from the individual.

The kit may be for performance of a mass spectrometry assay and may comprise a set of reference peptides (e.g. SRM peptides) in an assay compatible format wherein each peptide in the set is uniquely representative of each of GAPDH and the further marker proteins described provided in Table 1, Table 1 (A) Group 1; Table 1 (B) Group 2; or Table 1 (C) Group 3 or a combination thereof. Preferably two and more preferably three such unique peptides are used for each protein for which the kit is designed, and wherein each set of unique peptides are provided in known amounts which reflect the levels of such proteins in a standard preparation of said cell exposed to a known sensitizing compound. Optionally the kit may also provide protocols and reagents for the isolation and extraction of proteins from said cell, a purified preparation of a proteolytic enzyme such as trypsin and a detailed protocol of the method including details of the precursor mass and specific transitions to be monitored.

Optionally, the kits of the present invention may also comprise appropriate cells, vessels, growth media and buffers.

In a third aspect of the invention, there is provided a method for the diagnosis or prognostic monitoring of contact sensitizing by an allergen or irritant on an individual exposed to said allergen or irritant the method comprising determining the presence or level of expression of GAPDH and further marker proteinsselected from Table 1, Table 1 (A) Group 1; Table 1 (B) Group 2; or Table 1 (c) Group 3, in a mammalian cell obtained from said individual.

The mammalian skin cell is selected from the group consisting of a primary keratinocyte, a keratinocyte derived cell line, an epidermal keratinocyte, bulge-derived keratinocytes, foreskin keratinocytes, a human cell having keratinocyte properties, a cell derived from HaCaT cell line and a cell derived from NCTC2544 cell line..

The kit may also comprise printed instructions for performing the method.

In all aspects of the invention, the methods are *in vitro* methods carried out on a sample from a primary cell culture, an established cell line or a biopsy sample taken from a patient suffering from a contact allergy e.g. ACD, irritation. The sample used in the methods described herein may be a whole cell lysate, subcellular fraction e.g. cytoplasm, nucleus, mitochondria, cell membranes, cell culture medium supernatant, tissue or body fluid sample, for example a skin tissue sample, bronchoalveolar lavage (BAL) fluid, blood or a blood product (such as serum or plasma) sample or a urine sample.

In all aspects and in all embodiments of the invention, the further marker proteins are selected from Table 1, Table 1 (A) Group 1; Table 1 (B) Group 2; or Table 1 (c) Group 3 may be with the proviso that the marker is not one or more, or a combination of isoform 1 of Heat Shock cognate 71kDa protein; Protein S100-A8; Protein S100-A9; ADP/ATP translocase 2; Peptidyl- prolyl cis-trans isomerase A; Histone H2B type 1-L; Tubulin alpha-4A chain; protein disulfide-isomerase; Tu translation elongation factor mitochondrial precursor; Vimentin; SH3 domain binding glutamic acid-rich protein like 3; protein disulfide-isomerase A3; Annexin A5; Isoform 2 of Filamin-A; Galectin-1; Thioredoxin; Peroxiredoxin; Heat shock protein 90kDa alpha (cytosolic), class A member 1 isoform 1; Elongation factor 2; Fructose-bisphosphate aldolase A; Elongation factor 1- alpha; cDNA FLJ45706 fis, clone FEBRA2028457, highly similar to Nucleolin; and Isoform 2 of Nucleophosmin.

Embodiments of the present invention will now be described by way of example and not limitation with reference to the following accompanying figures. All documents mentioned herein are incorporated herein by reference.

### Brief Description of the Figures

**Figure 1****:** PLS loading plot of ANOVA filtered biomarkers found in human primary keratinocytes, wherein R2X[1] = 0,210301 R2X[2] = 0,224212.
**Figure 2****:** PLS score plot showing good separation between sensitizer and irritant exposed samples using biomarkers listed in Table 1.
**Figure 3****:** ELISA Quantification of Heat shock 70 kDa protein 1 (HSPA1A; HSPA1B) in keratinocytes
**Figure 4****:** Western Blot quantification of aldo-keto reductase C1 (AKRC1) in keratinocyte cell extracts
**Figure 5****:** Western Blot quantification of metallothionein 1G (MT1G) in keratinocyte cell extracts

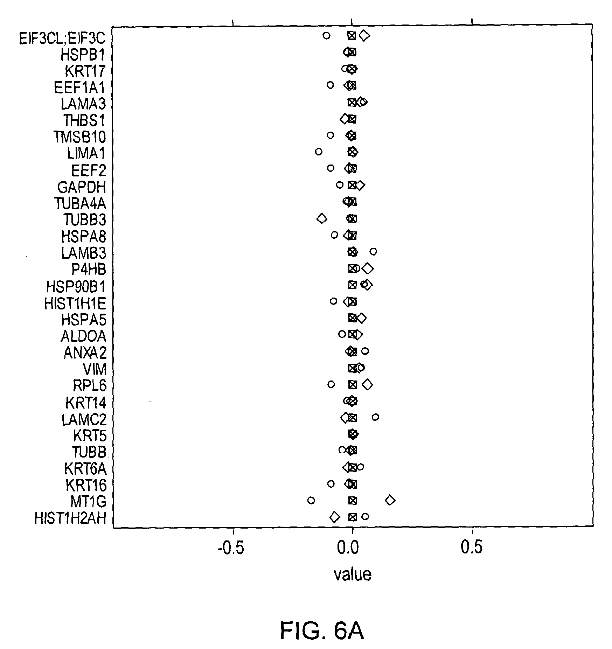

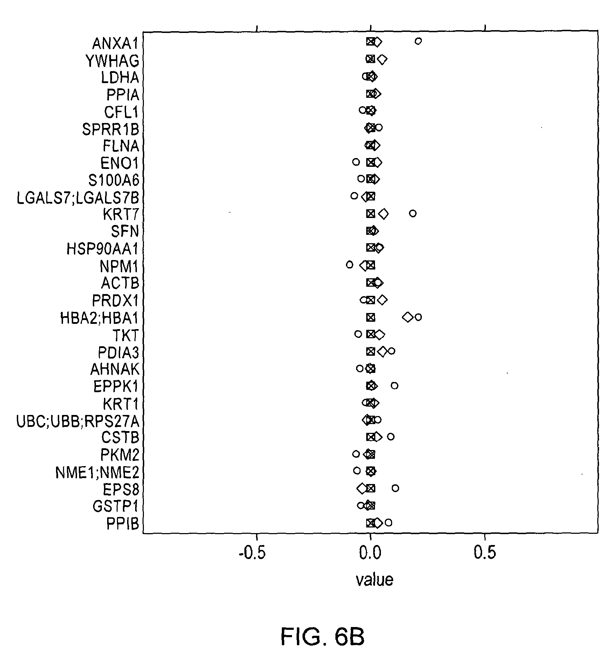

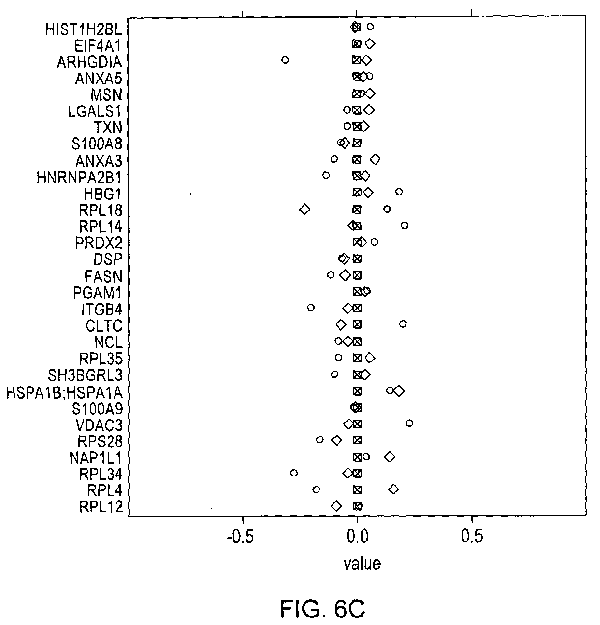

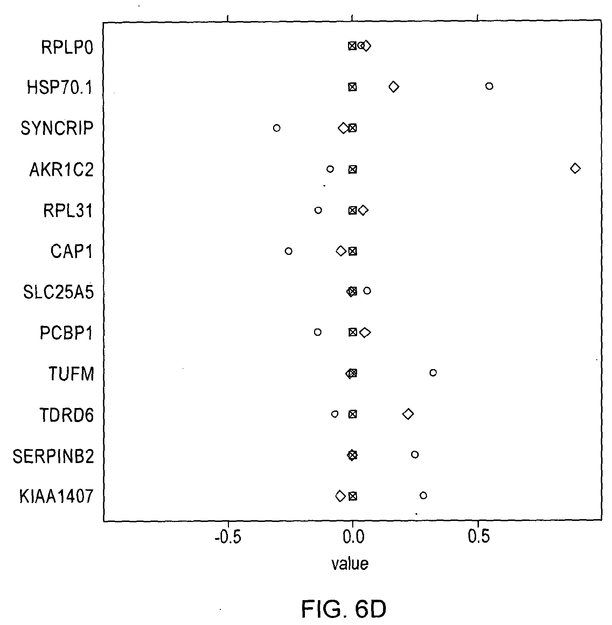

**Figure 6****:** Log2-transformed and referenced fold changes of 102 protein biomarkers Diamond: allergen; circle: irritant; rectangle: control.

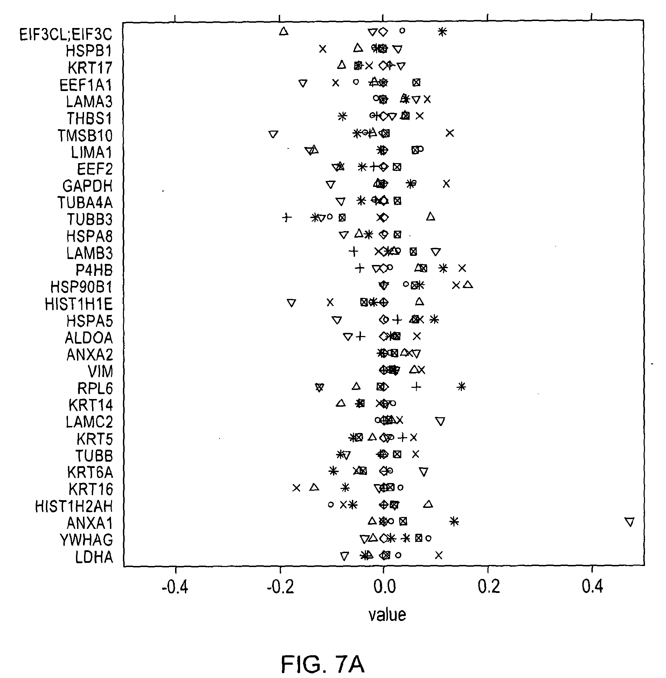

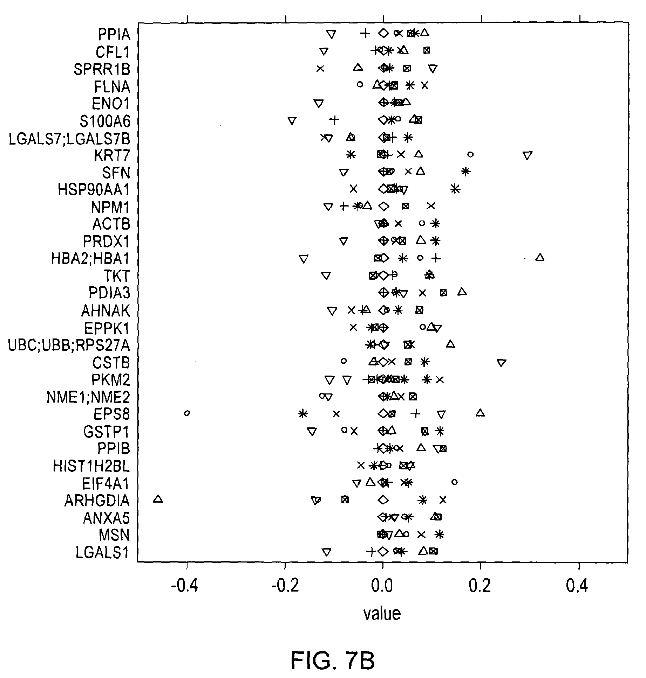

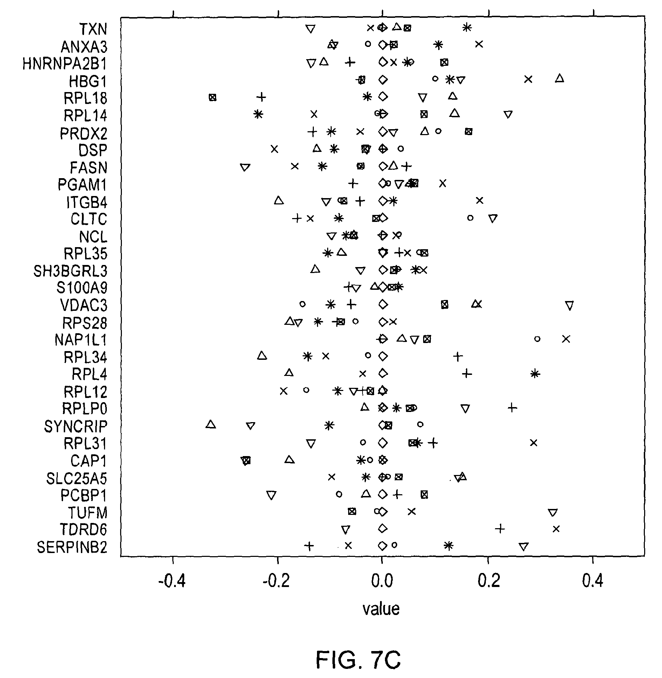

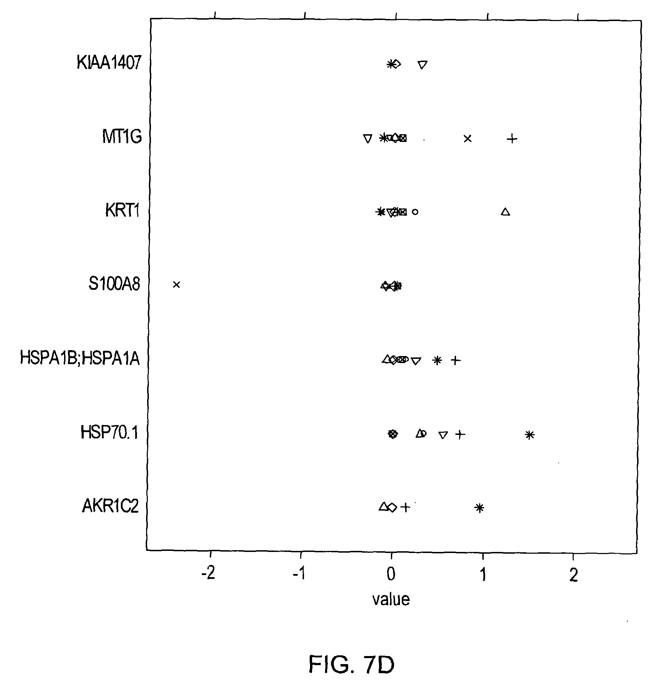

**Figure 7****:** Differences in the relative abundance of 102 protein biomarkers between test chemicals. Control: diamond; Nickel: cross; TMTD: plus sign; CA: asterisk; SDS: downward-pointing triangle; DNBS: square; DNCB: circle; SA: upward-pointing triangle.
**Figure 8****:** Table 4 - Protein Sequence Table.
**Figure 9****:** Table 5 - List of peptides, transition masses and mass spectrometer settings for TSQ Vantage (Thermo Scientific) used in the SRM assay

### Definitions

The term "antibody" includes polyclonal antiserum, monoclonal antibodies, fragments of antibodies such as single chain and Fab fragments, and genetically engineered antibodies. The antibodies may be chimeric or of a single species.

The term "marker protein" or "biomarker" includes all biologically relevant forms of the protein identified, including post-translational modification. For example, the marker protein can be present in a glycosylated, phosphorylated, multimeric or precursor form.

The term "control" refers to a cultured cell line, primary culture of cells taken from a human or animal subject, or biopsy material taken from a human or animal subject that has been incubated with an equivalent buffer to the test cells but lacking any test compound.

The terminology "increased/decreased concentration.. ..compared with a control sample" does not imply that a step of comparing is actually undertaken, since in many cases it will be obvious to the skilled practitioner that the concentration is abnormally high or low. Alternatively, the previously determined normal levels after exposure to non-sensitizing chemicals may be used as a reference value.

The term "antibody array" or "antibody microarray" means an array of unique addressable elements on a continuous solid surface whereby at each unique addressable element an antibody with defined specificity for an antigen is immobilised in a manner allowing its subsequent capture of the target antigen and subsequent detection of the extent of such binding. Each unique addressable element is spaced from all other unique addressable elements on the solid surface so that the binding and detection of specific antigens does not interfere with any adjacent such unique addressable element.

The term "bead suspension array" means an aqueous suspension of one or more identifiably distinct particles whereby each particle contains coding features relating to its size and colour or fluorescent signature and to which all of the beads of a particular combination of such coding features is coated with an antibody with a defined specificity for an antigen in a manner allowing its subsequent capture of the target antigen and subsequent detection of the extent of such binding. Examples of such arrays can be found at www.luminexcorp.com where application of the xMAP® bead suspension array on the Luminex® 100™ System is described.

The terms "selected reaction monitoring", "SRM" and "MRM" means a mass spectrometry assay whereby precursor ions of known mass-to-charge ratio representing known biomarkers are preferentially targeted for analysis by tandem mass spectrometry in an ion trap or triple quadrupole mass spectrometer. During the analysis the parent ion is fragmented and the number of daughter ions of a second predefined mass-to-charge ratio is counted. Typically, an equivalent precursor ion bearing a predefined number of stable isotope substitutions but otherwise chemically identical to the target ion is included in the method to act as a quantitative internal standard. Examples of such methods can be found at http://en.wikipedia.org/wiki/Selected_reaction_monitoring.

The term "sensitizer" means a chemical that induces an allergic response in exposed people or animals after repeated exposure to the chemical.

"Skin sensitization" means an immunological process which is induced when a susceptible individual is exposed topically to the inducing chemical allergen.

"Sensitizing potential" means the potential of a chemical compound or element to cause skin damage through topical exposure which may be by topical exposure. For the present purposes, the sensitizing potential of a compound includes its potential to cause damage via an allergic response (a sensitizer) and/or via inflammation (an irritant).

"Irritant" means a chemical that causes an inflammatory effect on living tissue by chemical action at the site of contact. It is important to include irritating chemicals when developing biomarkers for skin sensitization, because sensitizers (i.e. DNCB) can also exert irritation.

Chemicals which do not induce sensitization are referred to as "non-sensitizer", but may also include irritants.

### Detailed Description

The need for testing of chemical safety is a long established part of the regulatory process for pharmaceuticals and for the approval for sale of cosmetics and a wide range of other products that come into contact with human skin and mucosa. A number of testing regimes have been established and in some cases only a small number of these tests are proscribed as fit for purpose by national regulators. In the main these tests have been based on whole living organism studies, typically in rodent species.

There is now a strong ethical and economic driver to reduce the number of animals used in pharmaceutical and chemical safety testing and a concomitant need to find suitable *in vitro* tests to replace the proscribed testing methods. In this context we set out to demonstrate a set of proteins whose levels of expressions within a cultured cell line or tissue biopsy alter in a predictable manner in response to allergenic or irritant compounds.

To discover such a set of proteins we applied a proprietary proteomics discovery workflow to a human primary keratinocytes cultured in vitro. In brief, keratinocytes were cultured in the presence of known allergenic sensitizers, non-allergenic irritants or remained untreated. Keratinocytes were exposed to low and high dose of chemicals to look for dose effects. After exposure the keratinocytes were harvested and lysed and proteins extracted. Following extraction, the total cell lysate was subjected to proteolysis using trypsin and the resultant peptides labelled with one of a sixplex set of isobaric mass tags (Tandem Mass Tags®, Proteome Sciences plc).

Tandem Mass Tags are designed to allow the discriminant labelling of up to six different samples prior to mixing and analysis of all six samples in a single mass spectrometry experiment. Each tag in the set has the same overall mass (isobaric) but on fragmentation in the mass spectrometer releases a unique reporter ion whose intensity relative to the other reporter ions is directly proportional to the relative abundance of the protein in the sample. In our discovery experiments we were able to obtain relative quantitative information for 4245 peptides representing 1980 unique proteins consistently measured in at least 50% of all mass spectrometric measurements in a time- and cost-effective manner.

By allowing early mixing of samples the use of Tandem Mass Tags increases the robustness of the data allowing selection of the best candidates for subsequent routine measurement in a targeted screening test with higher throughput than discovery methods. However, to identify those proteins whose expression is predictably altered by chemical exposure it is necessary to undertake a panel of statistical analyses such as supervised and un-supervised cluster analysis. Through selective application of a range of such statistical tools we identified 102 protein markers that were significantly regulated in response to exposure to a set of training chemicals. Within these 102 proteins are markers of contact as well as non-sensitizing irritants.

Following the identification of candidate biomarkers using a set of training chemicals we developed a classification model that could predict whether a compound was a sensitizer/allergen or a non-sensitizing irritant based on the detected amount of one or more of the 102 biomarkers listed in Table 1. This classification model can be used to interpret the protein expression data from keratinocytes exposed to unknown chemicals or combinations of chemicals and to assign said chemicals into the allergen or irritant group. This test system is therefore suitable to replace living, whole-organism test for chemical safety.

It is recognised that the discovery methods used in this study are less well suited to the routine analysis of hundreds, thousands or tens of thousands of chemicals with unknown safety profile, such as will be needed to meet the ethical need to replace animal testing and the pending EU legislation. To overcome this potential bottleneck it will be necessary to provide more targeted means of analysis of one or more of the 102 protein biomarkers listed in Table 1. There are a number of suitable methods for the targeted measurement of up to 102 different proteins in a single analysis. One such technology is immunoassay where antibodies with specificity for the biomarker protein are used to capture, detect or capture and detect the protein. Immunoassay formats include but are not limited to enzyme-linked immunosorbent assay (ELISA), antibody sandwich ELISA, competitive ELISA, immunoPCR and Western blot. Where a small number of proteins are to be measured it is possible to use individual tests such as ELISA or western blot for each protein. Alternatively, multiplex testing methods such as antibody arrays and/or bead suspension arrays where a plurality of biomarkers are detected and quantified simultaneously can be used.

In some cases it may be undesirable or impossible to use antibodies to selectively quantitate levels of protein expression. Examples include where the biomarker is post-translationally modified as a result of chemical exposure and such modification is immunologically inert, or where proteolytic activity causes degradation of a protein thereby destroying epitopes recognised by available antibodies. In such situations non-antibody binding agents such as aptamers may be used. More preferably, quantitative mass spectrometry methods can be developed based on the principle of selected reaction monitoring (SRM).

In an SRM method peptides representing the target marker protein are selected based on empirical data obtained during marker discovery or are designed using in silico tools. Typically a combination of the two approaches is used for best results. For absolute quantitation by SRM it is necessary to provide an external equivalent 'heavy' peptide that is isotopically distinct to the native form to be measured in the analytical sample. There are a number of different approaches for the provision of such isotopically distinct reference peptides though they all share the common feature of adding one or more heavy stable isotopes into the peptide during production. The simplest approach which is often termed 'AQUA' is to use an amino acid containing one or more stable isotopes of hydrogen, carbon, nitrogen or oxygen. Typically a combination of isotopes is used to introduce a total mass difference of between 6 and 10 Daltons per peptide. There are a number of commercial sources of such heavy AQUA peptides (e.g. Thermo Scientific (www.thermoscientific.com)). An alternate to AQUA is to add heavy isotopes through a covalent label attached to a standard synthetic peptide. Such methods have the advantage of speed and cost of production of the reference peptides. However, the method then requires use of an isotopically distinct but chemically identical tag to label each analytical sample. There are a number of approaches for tag-based SRM methods including mTRAQ® (ABSciex) and TMT® (Thermo Scientific). Where multiple reference peptides are required it is possible to manufacture a synthetic gene encoding all desired peptides in a concatamer polypeptide. This is then transfected into a suitable expression host or *in vitro* transcription system and the expressed polypeptide purified prior to cleavage to release the individual reference peptides. Typically this would be performed using a heavy amino acid to provide the isotope substitution such that all peptides are 'heavy' relative to the natural form in the analytical sample. An example of such a method is the QCONCAT system (Pratt et al. Nature Protocols 1, - 1029 - 1043 (2006)).

Human keratinocytes were used as a skin keratinocyte cell culture model for developing a protein biomarker based *in vitro* assay system for determining the sensitizing potential of chemical sensitizers. It is consequently an additional aspect of the invention that these protein markers can also be used for the diagnosis of skin allergy in a mammal or human suspected of suffering from such an allergy. In this context a suitable tissue sample such as biopsy samples of skin are collected, proteins extracted and measured according to one of the methods of the present invention. The levels detected in the said sample are then compared with the levels known to be associated with a response to sensitizing agents in keratinocytes.

Four biomarker candidates HSPA8, S100A8, S100A9 and TYMB10 were chosen to develop a specific skin sensitization test for Nickel. In particular, the level of the three proteins HSPA8 and S100A9/A8 (synonyma: Calgranulin A/B; Calprotectin) is strongly and significantly altered by Nickel, whereas TYMB10 is used as a biomarker indicating if a chemical has the property of inducing skin irritation. Although Nickel is a common cause of allergic contact dermatitis in mice, it has been impossible to develop assays for this Nickel in mice (Kimber et al. 2011). The intended use of this assay is to determine the skin sensitization potential of Nickel salts in conjunction with testing the irritating potential of these chemicals in in vitro assays.

The invention is further illustrated by the following experiments.

### Example 1 - Proteomic analysis of a skin keratinocyte model

### Training chemicals used for the discovery of candidate biomarkers

A set of training chemicals was selected for biomarker discovery in human keratinocytes. The selected chemicals comprised 5 skin sensitisers of different strength, and 2 non-sensitisers/irritants (Table 2).

### Contact sensitizers/allergens:

DNCB: 1-chloro-2,4-dinitrobenzene (DNCB), is a organic compound used in colour photography processing. DNCB is considered an extreme allergen.
DNBS: 2,4-Dinitrobenzenesulfonic acid is yellow-tan powder used to prepare ether-soluble DNP derivatives of amino alcohols. DNBS is a strong contact sensitizer.
Cinnamic aldehyde: 3-phenyl-2-Propenal; Cinnamal, Cinnamaldehyde is an oily yellow liquid with strong odor of cinnamon. This compound is the main component of cinnamon oil. The predominant application for cinnamaldehyde is in the flavour and fragrance industries. It is used as a flavouring for chewing gum, ice cream, candy, and beverages. Cinnamic aldehyde is considered a moderate sensitizer.
TMTD: Tetramethyl thiuram disulfide, thiram, thiuram, or Sovchem® is white powder or granule. TMTD is a fungicide used a seed protectant and to protect fruit, vegetable, crops from a variety of fungal diseases and from damage by rodents and rabbits. TMTD is moderate sensitizer.
Nickel: Nickel is a silvery-white metal that is mixed to produce alloys. It is used to manufacture stainless steel, coins, body piercings or jewellery. Although Nickel is a weak sensitizer it is a common course of contact allergy.

### Irritating/non-sensitizing chemicals:

SDS: Sodium dodecyl sulfate (SDS), lauryl sulfate (SLS) or sodium laurilsulfate is an anionic surfactant used in many cleaning and hygiene products. SDs can cause skin and eye irritation.
Salicylic acid: is also known as 2-hydroxybenzenecarboxylic acid. Salicylic acid is known for its ability to ease aches and pains and reduce fevers. Salicylic acid is a key ingredient in many skin-care products for the treatment of acne, psoriasis, calluses, corns, keratosis pilaris, and warts. Because of its effect on skin cells, salicylic acid is used in several shampoos used to treat dandruff. Exposure to salicylic acid can cause hypersensitivity.

### Cell culture

Primary human keratinocytes were obtained from Provitro (Berlin, Germany). Cells were seeded at 3 x 10E5 cells per 10 cm dish. Cells were grown in serum free supplemented keratinocyte growth medium (Provitro) to 80-90% density prior to treatment. Cells were grown at 37 °C in 5% CO2. Second and third passages post-cryopreserved seedings were used for experiments

### Chemical exposure of cells

**Table 2: List of reference chemicals used to discover biomarkers**

| Chemical | Chemical/ Solvent | Final test concentration | | |
|---|---|---|---|---|
| | | Low | High | LLNA EC3 (%) |
| 2,4-Dinitrobenzenesulfonic acid (DNBS) | DNBS in DMSO | 4 µM | 8 µM | 1.98 (strong) |
| Dinitrochlorobenzene (DNCB) | DNCB in DMSO | 4 µM | 8 µM | 0.05 (extreme) |
| Cinnamic aldehyde (CA) | CA in DMSO | 38 µM | 136 µM | 2.8 (moderate) |
| Tetramethylthiuram disulfide (TMTD) | TMTD in DMSO | 8.3 µM | 13.3 µM | 5.2 (moderate) |
| Nickel sulfate | Ni in saline | 250 µM | 1.500 µM | False negative |
| Sodium dodecyl sulphate (SDS) | SDS in saline | 49 µM | 60 µM | 14 (False positive) |
| Salicylic acid (SA) | SA in DMSO | 250 µM | 5000 µM | Non-sensitizer |

Test chemicals were added and the plates were incubated for 24 hours at 37°C in a 5% CO2 humidified incubator. When a chemical was dissolved in DMSO, a final concentration of 0.1% DMSO was used in the relevant negative control. After 24 h incubation, cells were harvested and washed twice in PBS.

### Cell lysis and sample preparation

Cells were lysed in four volumes of 100 mM TEAB (triethylammonium bicarbonate), pH 8.5+0.1, 1 mM TCEP (tris[2-carboxyethyl]phosphine*HCl), 0.1 % SDS. After suspending the cell pellet in lysis buffer the suspension was heated at 95°C for ten minutes in a thermomixer ([Eppendorf, Thermomixer comfort). Cell lysates were sonicated twice on ice for two minutes followed by a second cycle heating and sonication. Samples were then centrifuged at 14.000 g for ten minutes and supernatants were used for further analyses or stored at-80°C.

### Determination of protein concentrations

The protein concentration was determined using the Bradford reagent. The results were calculated using a standard curve created from measurements of dilutions of a standard consisting of BSA/IgG (50%/50%).

### Trypsin digestion and isobaric mass tag labelling

Tandem Mass Tags (TMTs) (Thermo Scientific) comprise a set of amine-reactive isobaric labels, which are synthesized with heavy and light isotopes to present the same total mass but to provide reporter-ions at different masses after activation with collision-induced dissociation (CID) and subsequent tandem mass spectrometry (MS/MS).

Equivalents of up to 100 µg protein solution per sample were used for proteomics profiling experiments. A reference pool was created from an aliquot of all samples and included in each TMTsixplex labelling reaction. The final volume of the sample was adjusted with TMT labelling buffer (100mM TEAB pH 8.4-8.6, 0.1% SDS) to 100 µl per sample. The samples were reduced for 30 min at room temperature by the addition of 5.3 µL each of 20 mM TCEP in water and subsequently alkylated for 1 h at room temperature by the addition of 5.5 µL each of 150 mM iodoacetamide in acetonitrile.

For protein digestion, 10 µL of a 0.4µg/µL trypsin solution (sequencing grade modified trypsin, Promega) in 100mM TEAB buffer pH 8.4-8.6 was added to each vial and incubated at 37°C for 18 h. The digested protein samples were labelled with the TMTsixplex reagents TMT6-126, TMT6-127, TMT6-128, TMT6-129, TMT6-130 and TMT6-131). TMTsixplex reagents were dissolved in acetonitrile to yield a concentration of 60mM and 40.3 µL of the corresponding reagent solution were added to the sample vials and samples incubated for 1h at room temperature. To reverse occasional labelling of Tyr, Ser and Thr residues, 8 µL of an aqueous hydroxylamine solution (5% w/v) was added and incubated for 15 min at room temperature. The TMTsixplex-labelled samples were combined and purified.

### Sample purification

The samples were diluted with 3 mL water/acetonitrile 95:5 + 0.1% TFA each and desalted using HLB Oasis cartridges (1cc, 30mg, Waters). The eluate fraction each was further purified by strong cation exchange using self-made cartridges (CHROMABOND empty columns 15ml, Macherey-Nagel, filled with 650µL SP Sepharose Fast Flow, Sigma). After loading the peptides and washing with 4 mL water/acetonitrile 75:25 + 0.1% TFA, the peptides were eluted with 2 mL H₂O:ACN 75:25 + 400mM ammonium acetate. The samples were dried in a vacuum concentrator and dissolved in 50 µL water/acetonitrile 95:5 + 0.1% TFA each and stored at -20°C until analysis.

### Liquid chromatography and tandem mass spectrometry (LC-MS/MS)

The TMTsixplex labelled samples were measured by High-Performance Liquid Chromatography-Tandem Mass Spectrometry (HPLC-MS/MS). For example, 5 µL (5 µg) of each sample were injected and measured using an electrospray ionization linear ion trap quadrupole Orbitrap mass spectrometer (Thermo Scientific) operated in CID (collision-induced dissociation) and HCD (higher collision decomposition) mode. The peptide mixture was separated on an Eksigent NanoLC using a Reprosil C18 trapping column (10 x 0.1 mm, 5 um) and a Reprosil C18 analytical column (400 x 0.075 mm, 3 um) at a flow rate of 350 nL/min (60 min gradient: 5-30 % acetonitril). Instrument parameters were: Resolution (MS = 30,000; MS/MS = 7,500), collision energy (CID =35 and HCD =75) isolation width (CID=2 u, HCD=1.5 u).

Peaks lists were generated from Orbitrap raw data files as mascot generic files (*.MGf data files) using Proteome Discoverer (version1.1; ThermoFisher, San Jose, USA). The resulting *.mgf files were searched against the IPI human database (version 3.68 from February 2011) by MASCOT (version 2.2; MatrixScience, London, UK (Probability-based protein identification by searching sequence databases using mass spectrometry data. Perkins DN, Pappin DJ, Creasy DM, Cottrell JS. Electrophoresis. 1999 Dec;20(18):3551-67.)). Peptide and protein identification was performed using the following parameters:
Carbamidomethyl at Cysteines and TMT modifications at N-terminal site and at Lysines were set as fixed modifications. Trypsin was used for the enzyme restriction, with three allowed miscleavages and an allowed mass tolerance +/-10 ppm for the precursor masses and 0.05 for the fragment ion mass. The corresponding MASCOT result files (*.dat data file) were downloaded and reporter ion intensities and protein identifications were extracted with an in-house tool. Reporter ion intensities and protein identities were exported into a relational MySQL database (version 5.157; Oracle, Redwood Shores, USA) and log2 ratios of reporter ions were calculated.

### Data pre-processing of extracted reporter ion intensities and relative quantitation

The six reporter ion intensities of the isobaric mass tags were corrected for isotopic distribution and systematic bias by means of sum scaling based on the assumption of a constant integral of any reporter ion series within one LC/MS/MS run. In addition, those MS/MS scans were filtered out where the reporter ion intensity of all six tags was smaller than 80 AU (arbitrary units) and where the reporter ion intensity of less than two tags was smaller than 10 AU. The relative intensities of reporter ions represent the relative amount of a peptide in the sample. To compare the relative amount of a peptide to all samples, a ratio is calculated between each sample versus the pooled reference sample. In some experiments each sample a ratio was calculated between each sample and a respective control sample generated from the same donor. The ratio was log2 transformed to yield referenced measurement values for each peptide. To obtain information on relative changes on the protein level, the log2 reference reporter ion intensities for each identified peptide belonging to one protein identity were averaged as the geometric mean.

### Example 2 - Statistical analysis and generation of a classification model

Reference chemicals belonging to the groups of sensitizer and irritant as well as appropriate (vehicle) controls were used to select candidate protein biomarkers. These chemicals were applied in two different concentrations to look for concentration dependent effects.

For multiple hypothesis testing an analysis of variance (ANOVA, p<=0.05) was computed to investigate biomarkers related to any of the possible contrasts between the three classes. To account for variance introduced from analysing different donors and biological replicates linear mixed- effects models (LME) were used to identify biomarker. The statistical scripting language R or the data analysis software MeV (TIGR) version 4.3 was used for all statistical analyses.

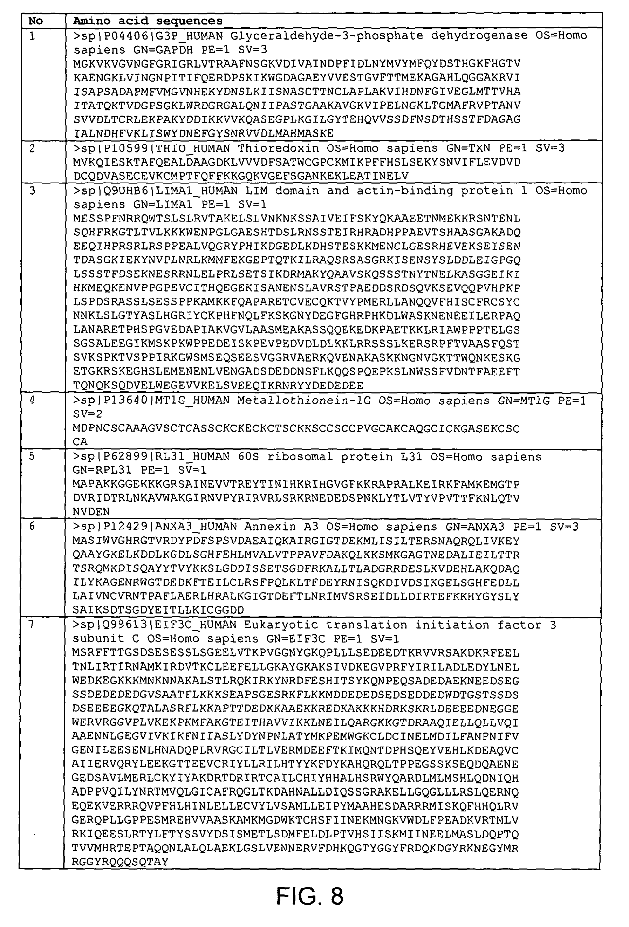

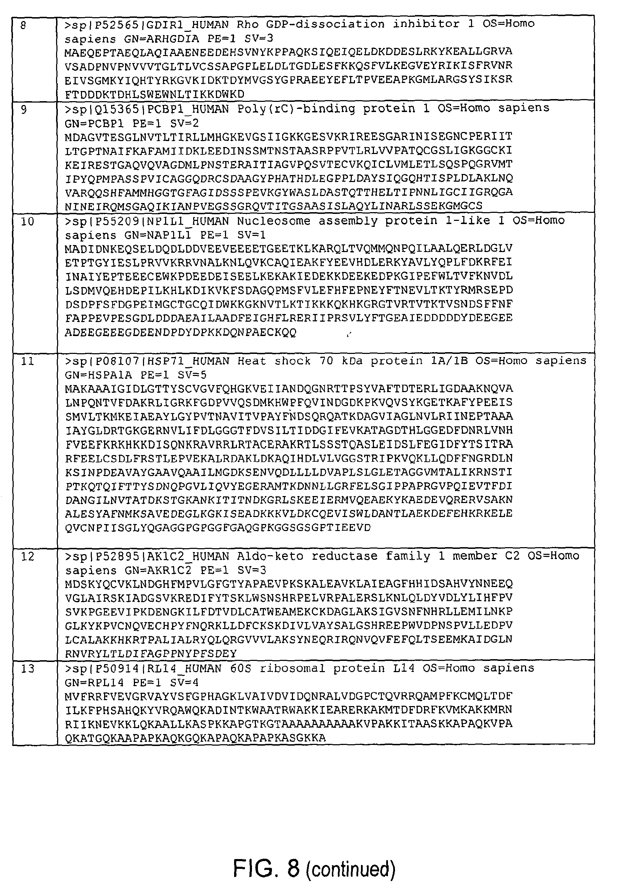

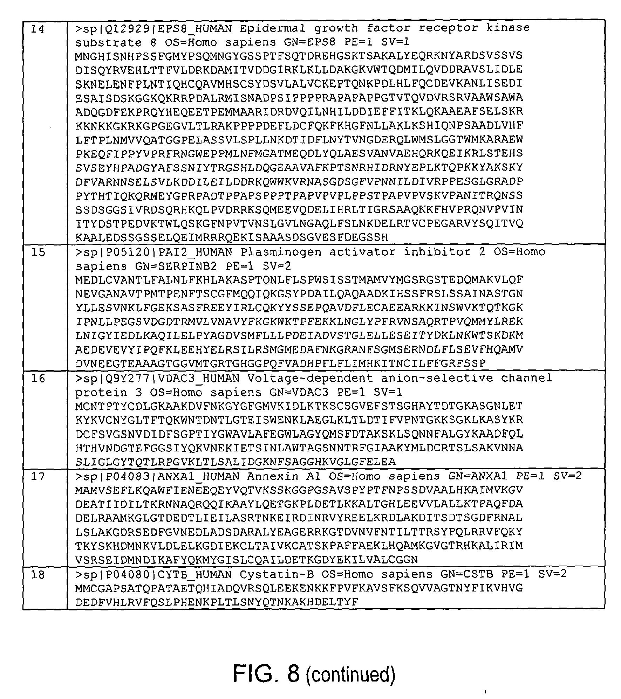

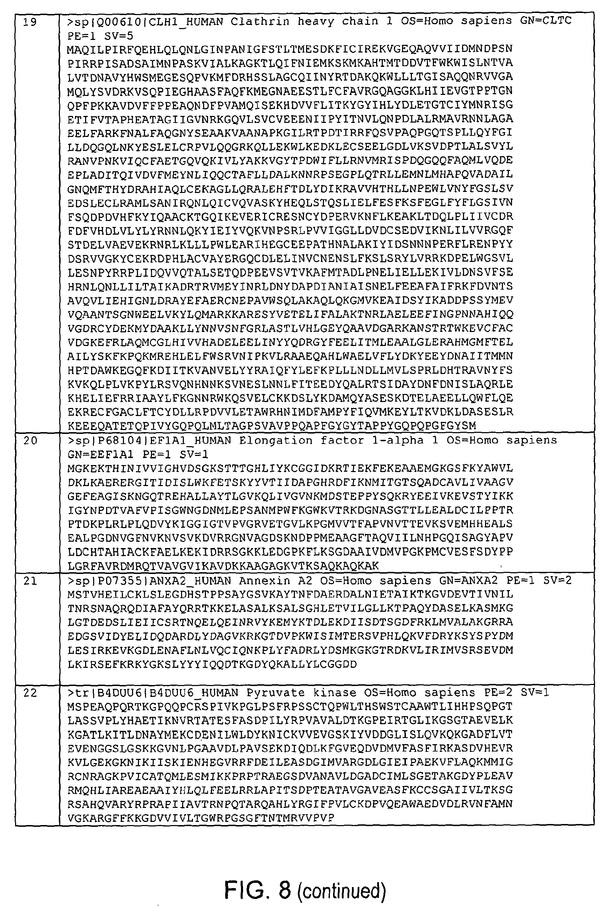

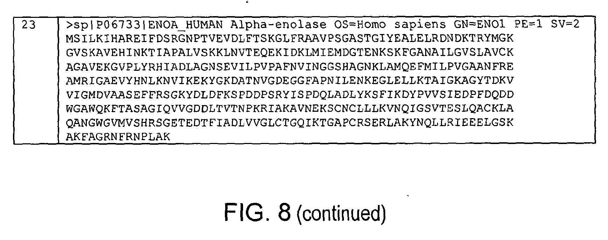

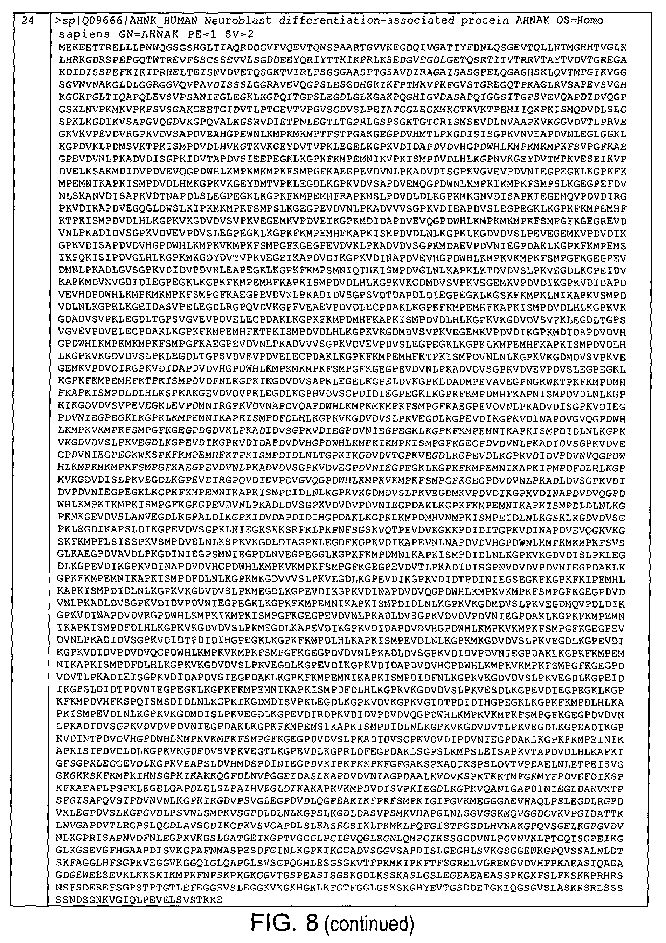

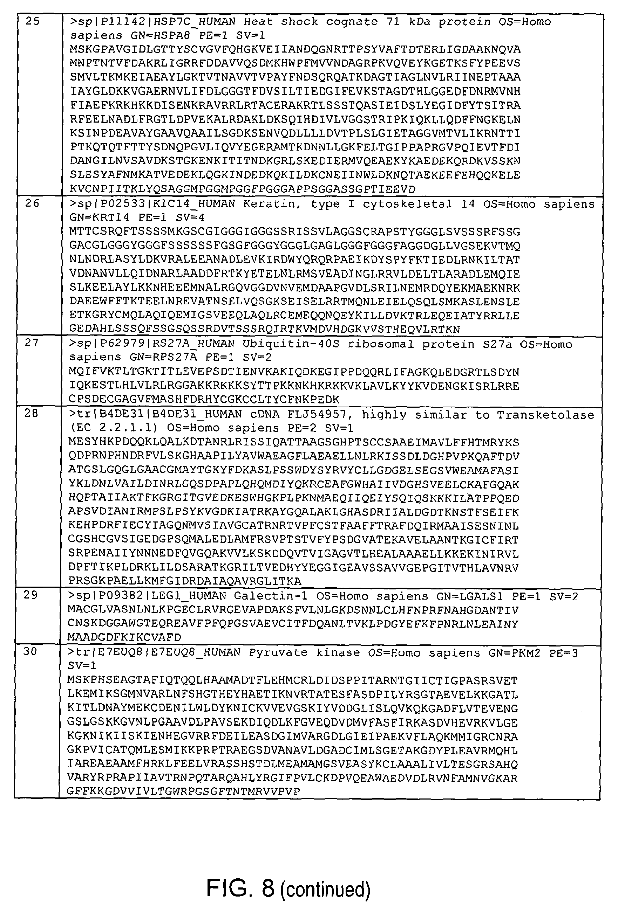

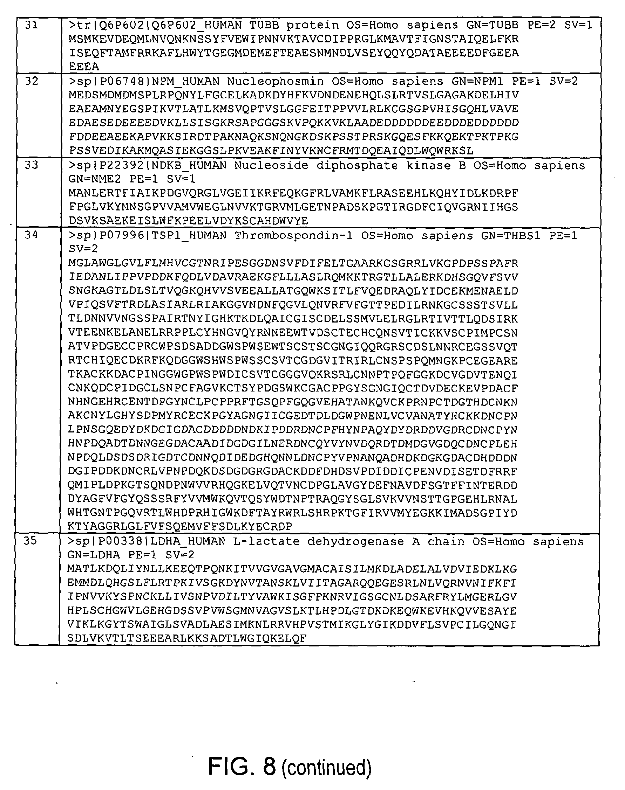

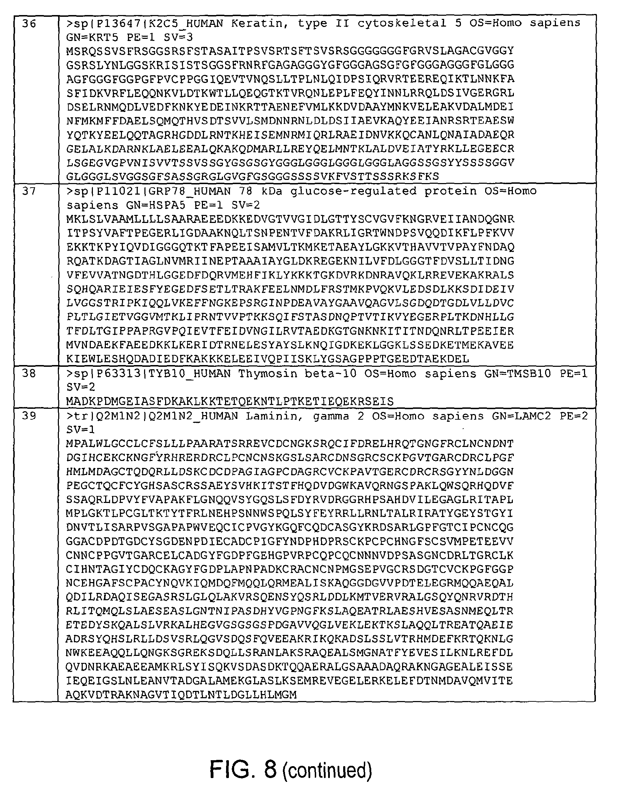

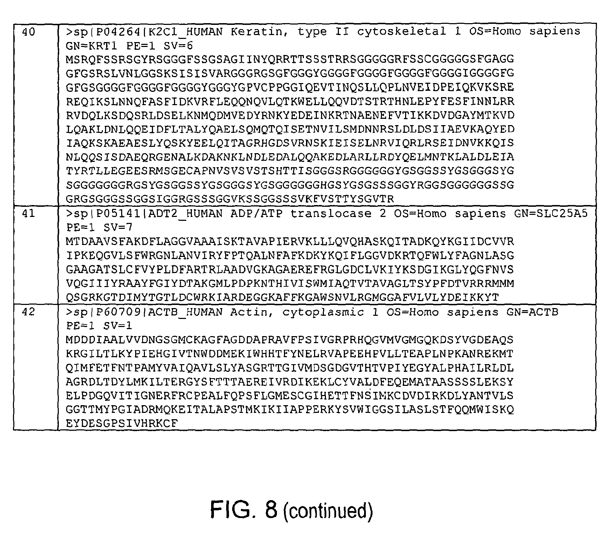

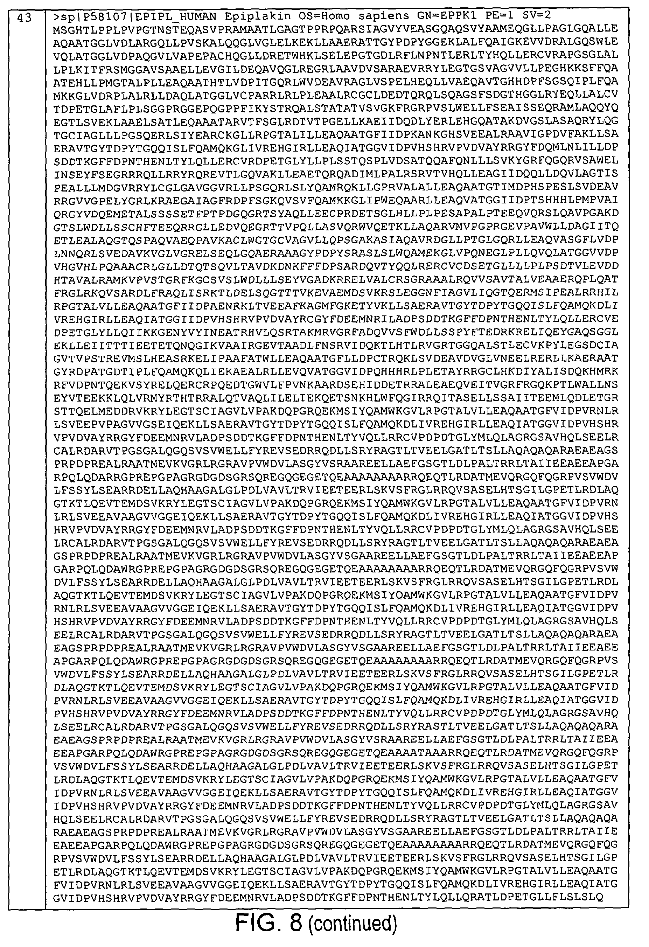

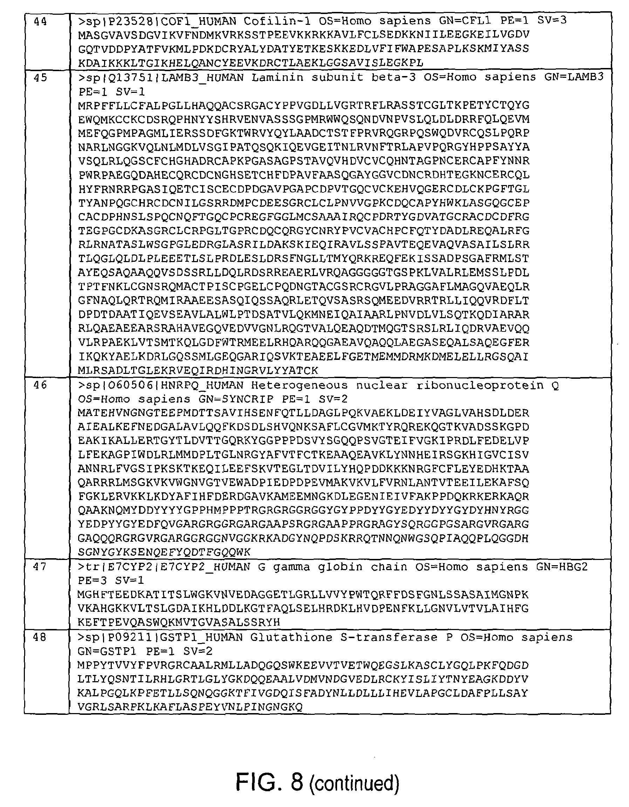

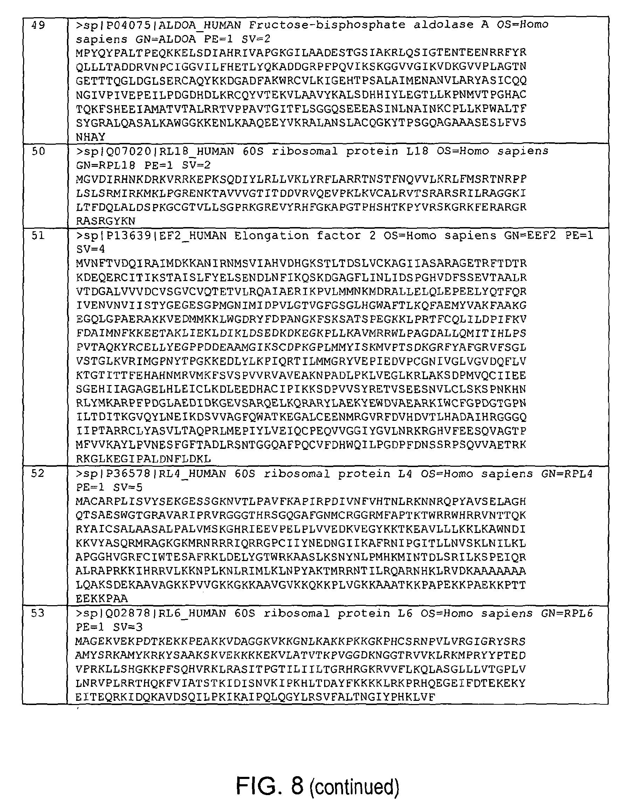

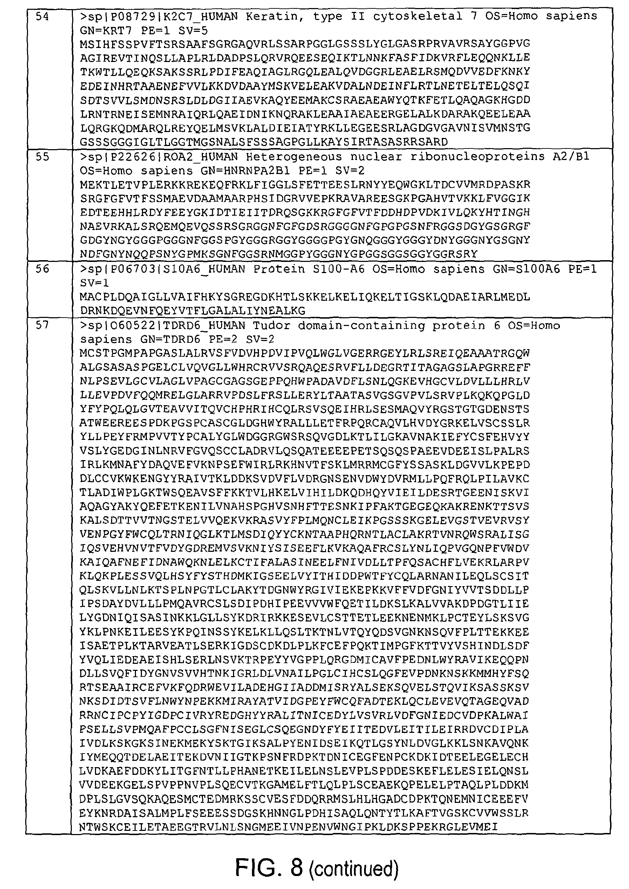

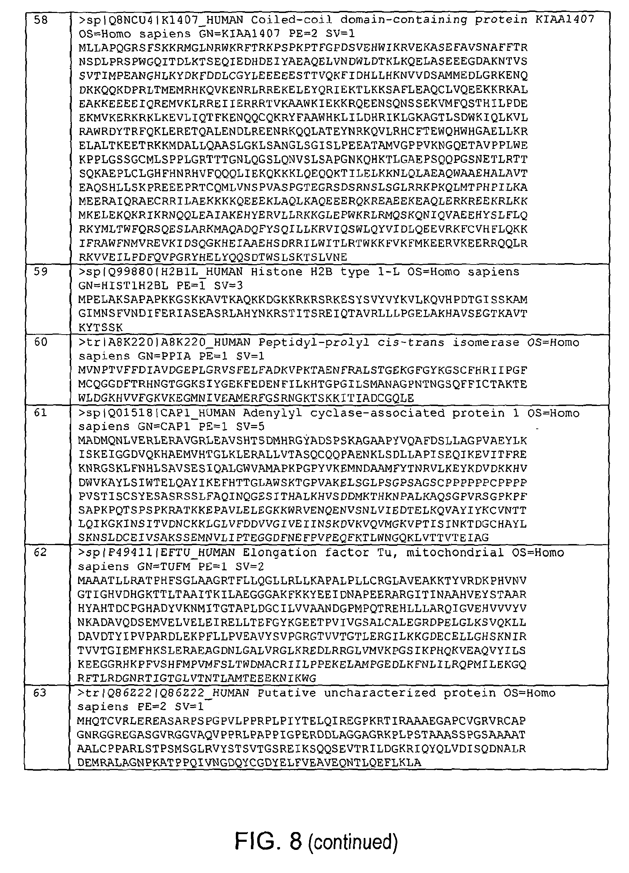

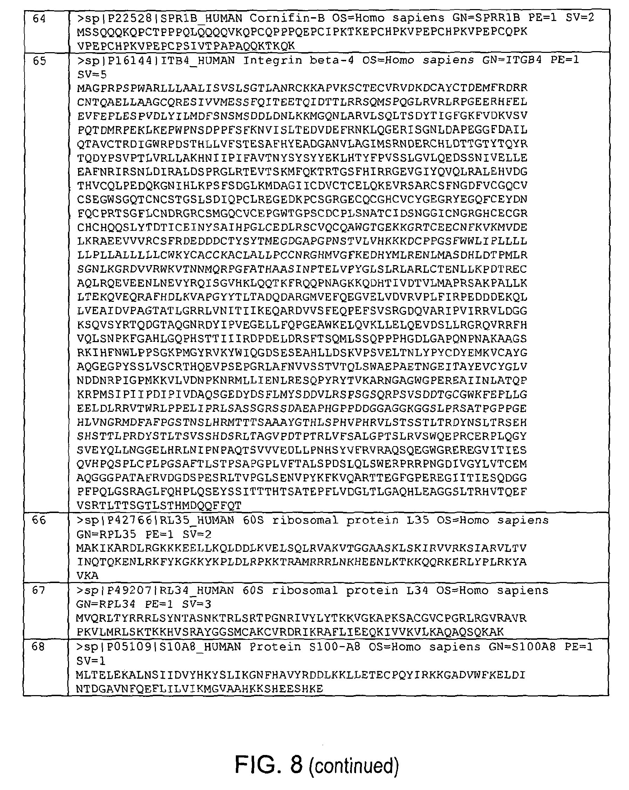

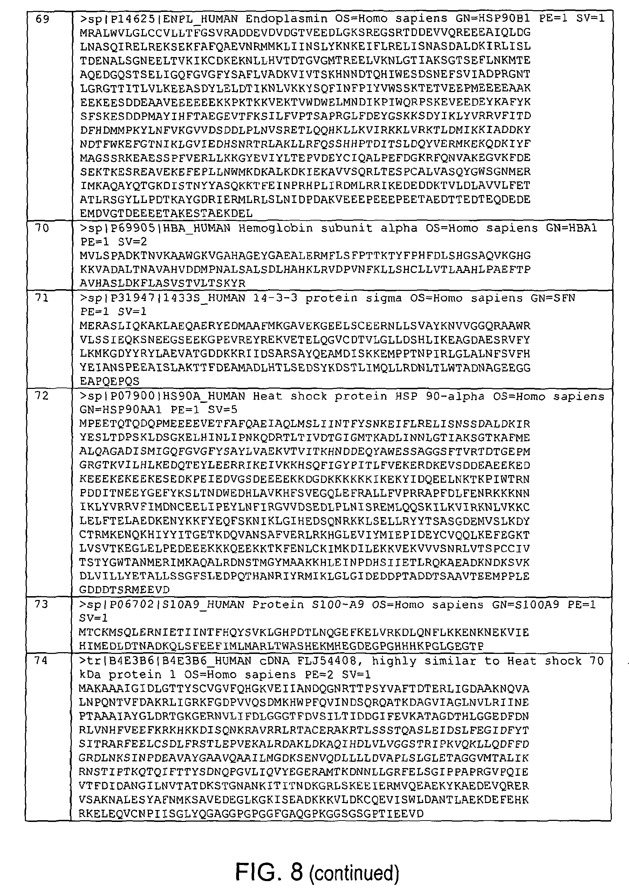

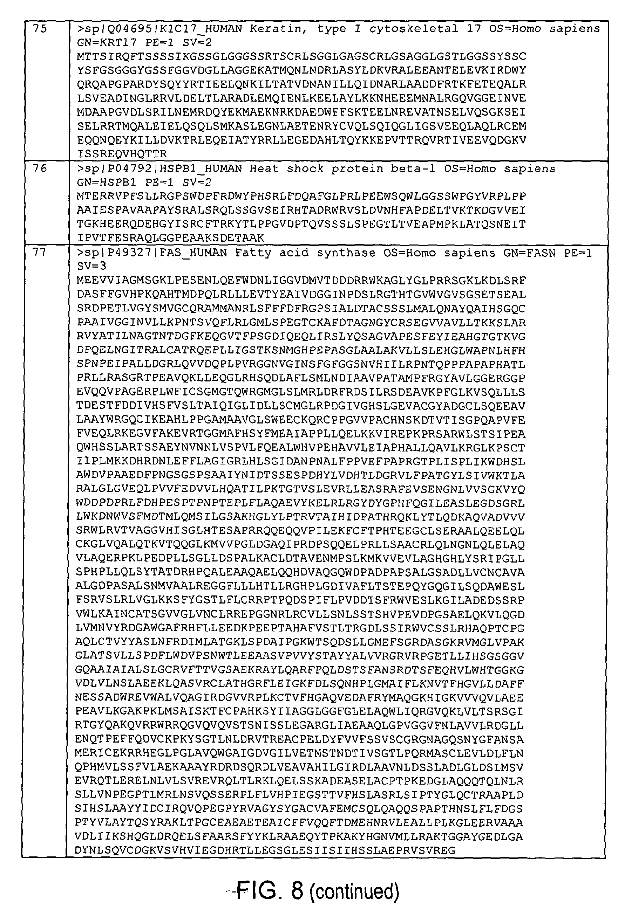

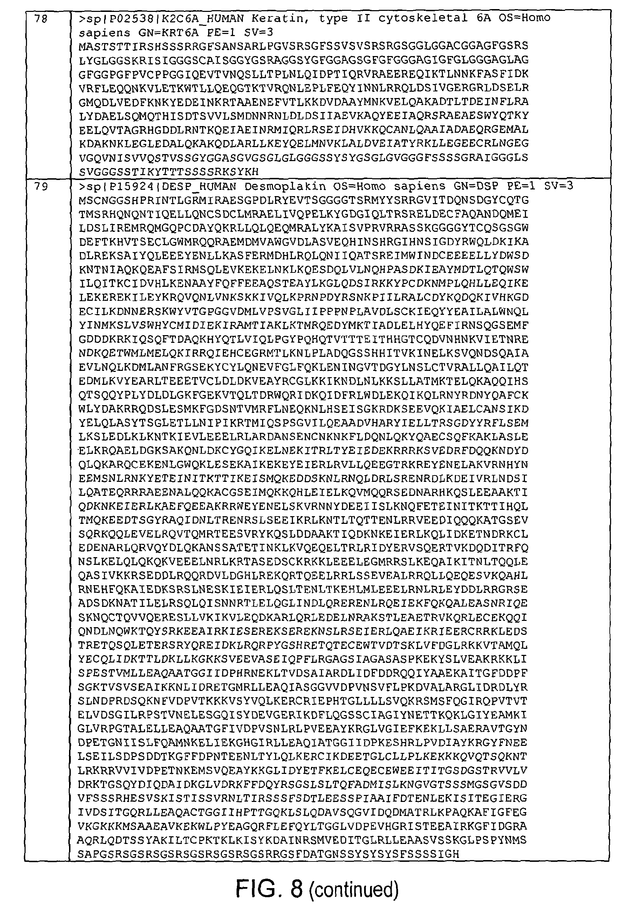

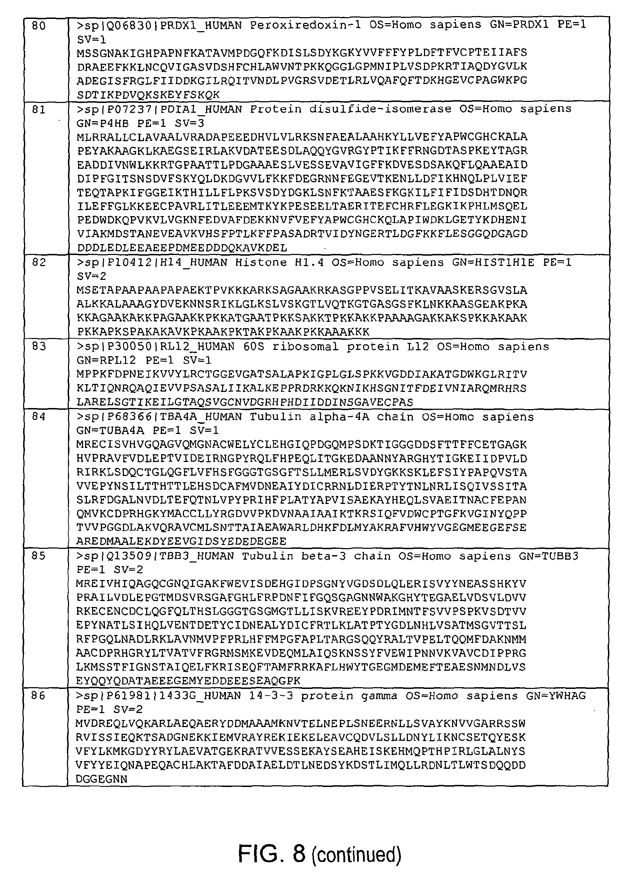

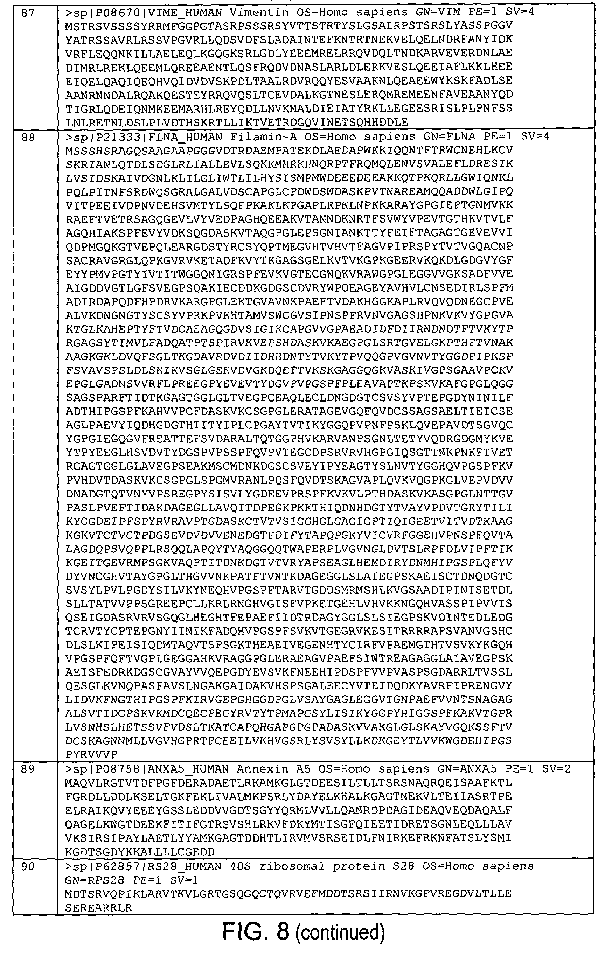

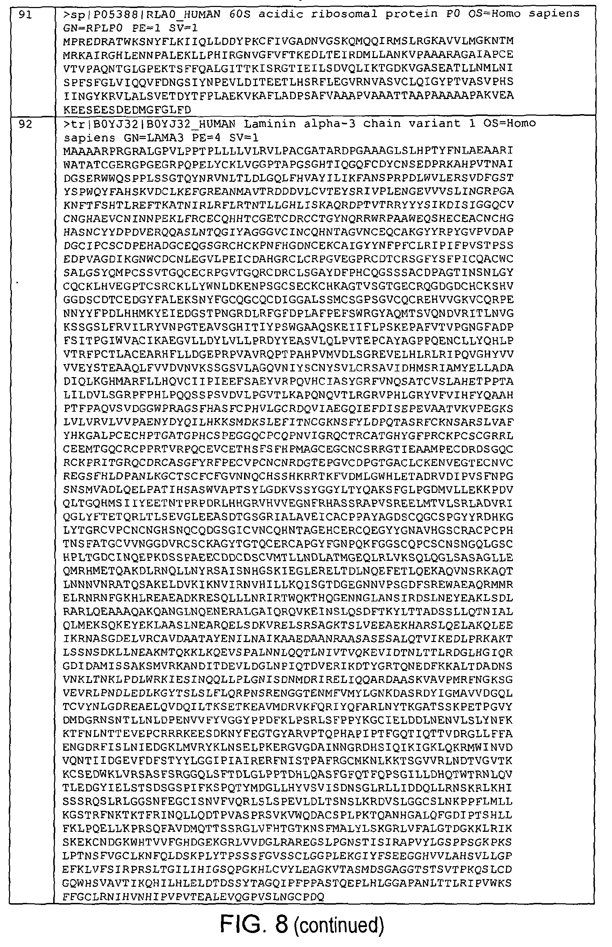

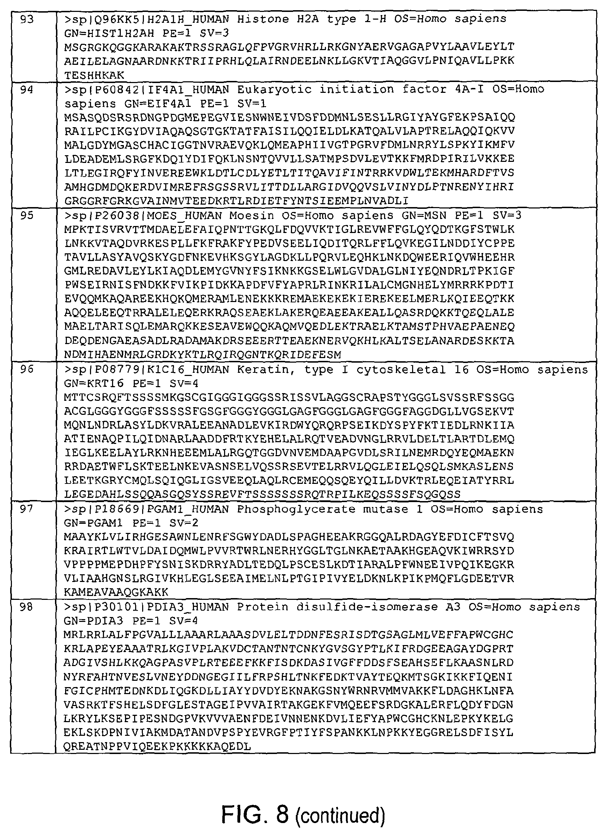

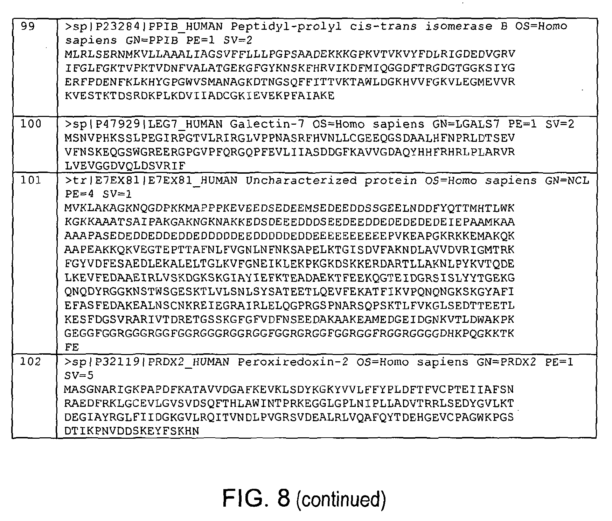

Thereafter, a list of 102 protein biomarker was obtained (No. 1 to 102). The list is detailed in Table 1 and in the protein sequence Table 4 (Figure 8).

Identification of candidate protein biomarkers for the assay To discover candidate protein biomarkers that are allow discriminating between sensitizer and non-sensitizer two four discovery studies comprising training chemicals listed in Table 2 were performed as described below.

In the first study keratinocytes obtained from donor were incubated with two irritants 45 µM SDS, 60 µM SDS, 250 µM SA and 500 µM SA) 5 sensitizers (38 µM CA, 136 µM CA, 4 µM DNBS, 8 µM DNBS, 4 µM DNCB, 8 µM DNCB, 8.3 µM TMTD, 13.3 µM TMTD, 250 µM Nickel and 1500 µM Nickel) and controls in two concentrations. Duplicate analyses were performed.

In the second study keratinocytes from four different donors were incubated with two irritants 45 µM SDS, 60 µM SDS, 250 µM SA and 500 µM SA) 5 sensitizers (38 µM CA, 136 µM CA, 4 µM DNBS, 8 µM DNBS, 4 µM DNCB, 8 µM DNCB, 8.3 µM TMTD, 13.3 µM TMTD, 250 µM Nickel and 1500 µM Nickel) and controls in two concentrations. Duplicate analyses of each donor were performed yielding a total number of 8 samples per chemical.

The aim of the statistical analysis was to develop a classification model which allows assignment of a chemical into the group of sensitizing chemicals (A= allergen) or non-sensitizing chemicals (I= irritant). After statistical analysis of the two data sets (p<=0.05) a final list of candidate biomarkers was obtained. Each of the two data sets involved testing of chemical allergens of different sensitizing potency as well as the metal ion nickel which gave false negative results in the LLNA. Thus, the use of the methods of the present invention for assessment of new chemicals or analyzing different combinations of chemicals will contribute to a growing database of biomarker candidates. In a first approach the most appropriate candidates were top- down ranked based on increasing p-value. Table 1 shows the top 102 candidate biomarkers that were significantly influenced after exposure of keratinocytes to a set of training chemicals.

Consequently Sensitizer (A=allergen) and non-sensitizer (I=irritant) can be identified by measuring the abundance of a very limited set of gene products expressed in keratinocytes or keratinocyte cell lines:
GAPDH, TXN, LIMA1, MT1G, RPL31, ANXA3, EIF3CL;EIF3C, ARHGDIA, PCBP1, NAP1L1, HSPA1B;HSPA1A, AKR1C2, RPL14, EPS8, SERPINB2, VDAC3, ANXA1, CSTB, CLTC, EEF1A1, ANXA2, PKM2, ENO1, AHNAK, HSPA8, KRT14, UBC;UBB;RPS27A, TKT, LGALS1, PKM2, TUBB, NPM1, NME1;NME2, THBS1, LDHA, KRT5, HSPA5, TMSB10, LAMC2, KRT1, SLC25A5, ACTB, EPPK1, CFL1, LAMB3, SYNCRIP, HBG1, GSTP1, ALDOA, RPL18, EEF2, RPL4, RPL6, KRT7, HNRNPA2B1, S100A6, TDRD6, KIAA1407, HIST1H2BL, PPIA, CAP1, TUFM, SH3BGRL3, SPRR1B, ITGB4, RPL35, RPL34, S100A8, HSP90B1, HBA2;HBA1, SFN, HSP90AA1, S100A9, HSP70.1, KRT17, HSPB1, FASN, KRT6A, DSP, PRDX1, P4HB, HIST1H1E, RPL12, TUBA4A, TUBB3, YWHAG, VIM, FLNA, ANXA5, RPS28, RPLP0, LAMA3, HIST1H2AH, EIF4A1, MSN, KRT16, PGAM1, PDIA3, PPIB, LGALS7;LGALS7B, NCL, PRDX2

In a second approach linear mixed-effects models (LME) were used to account for between-donor components of variance for each biomarker in this study. LME yielded a subset of 47 protein biomarker related to sensitizer/allergen exposure and a subset of 46 biomarkers related to non-sensitizer/irritant exposure.

The list of allergen-specific biomarkers identified using LME comprises:
GAPDH, TXN, MT1G, NAP1L1, HSPA1B;HSPA1A, AKR1C2, ANXA1, EEF1A1, PKM2, LDHA, KRT5, HSPA5, ACTB, RPL18, EEF2, PPIA, S100A8, HSP90B1, HBA2;HBA1, SFN, HSP90AA1, S100A9, HSP70.1, KRT17, HSPB1, FASN, KRT6A, DSP, PRDX1, P4HB, HIST1H1E, RPL12, TUBA4A, TUBB3, YWHAG, VIM, FLNA, ANXA5, RPS28, RPLP0, LAMA3, HIST1H2AH, EIF4A1, MSN, KRT16, PGAM1, PDIA3

The list of irritant-specific biomarkers identified using LME comprises:
GAPDH, TXN, ARHGDIA, PCBP1, HSPA1B;HSPA1A, RPL14, SERPINB2, VDAC3, ANXA1, CSTB, EEF1A1, ANXA2, PKM2, ENO1, AHNAK, HSPA8, KRT14, UBC;UBB;RPS27A, TUBB, NPM1, NME1;NME2, LDHA, KRT5, TMSB10, LAMC2, KRT1, SLC25A5, CFL1, LAMB3, HBG1, GSTP1, EEF2, RPL6, KRT7, S100A6, HSP90B1, HSP70.1, KRT17, TUBA4A, RPS28, KRT16, PDIA3, PPIB, LGALS7;LGALS7B, NCL, PRDX2

### Partial Least Squares - Discriminant Analysis (PLS-DA)

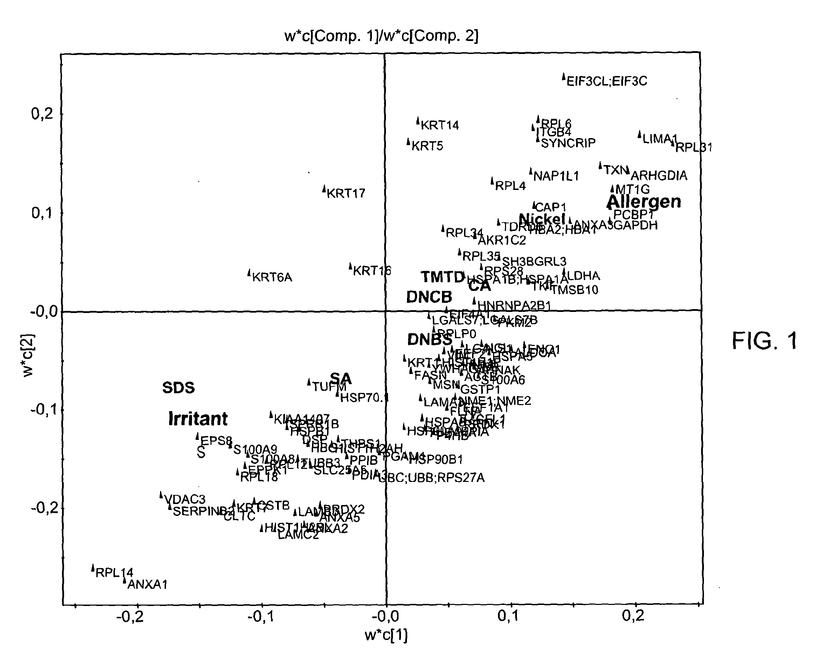

It is also possible to conduct a Partial Least Square Regression (PLS) analysis to select the most promising proteins by looking at the loadings scores of the two first latent components (Figure 1). A model was built using the response variables (y) "allergen" and "irritant" and the ANOVA and LME filtered proteins as predictors (x). The first PLS components (x-axis) was plotted against the second PLS component (y-axis). Biomarkers that are close to the response variable "allergen" have a strong role in classifying chemicals as potential allergens. Biomarkers that are close to the response variable "irritant" identify chemicals as irritants. Based on the PLS loading plot (Figure 1) the 12 most important proteins for identifying allergens comprises proteins of Group 1 (Table 1). Group 2 (Table 1) comprises proteins most important for identifying irritant/non- sensitizer.

### Group 1:

GAPDH, TXN, LIMA1, MT1G, RPL31, ANXA3, EIF3CL; EIF3C, ARHGDIA, PCBP1, NAP1L1, HSPA1B; HSPA1A, AKR1C2

### Group 2:

RPL14, EPS8, SERPINB2, VDAC3, ANXA1, CSTB

### Group 3:

Group 3 of Table 1 contains further sensitizer biomarkers that pass the required significance criteria of p<=0.05 in at least one of the four studies that involved testing of different combinations of chemical irritants and sensitizers.

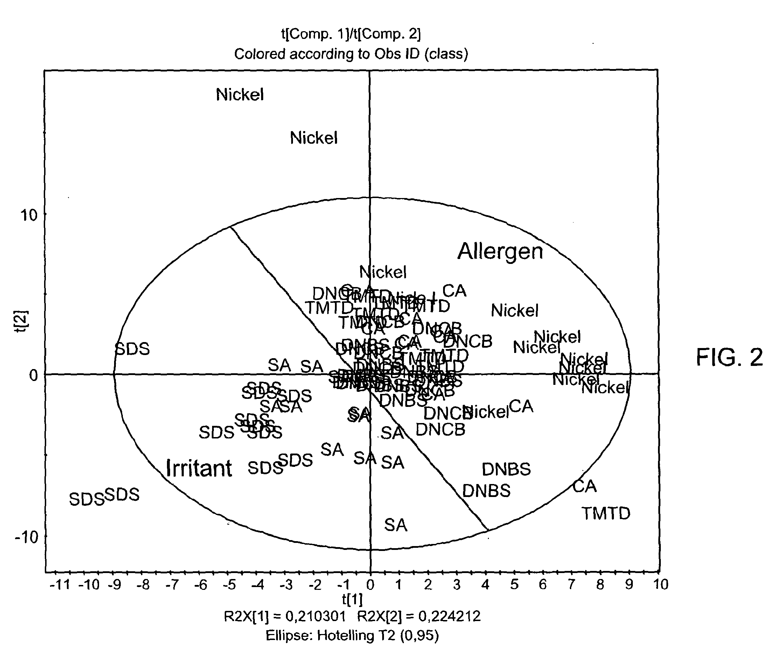

A preferred method for classification of chemicals is employing PLS analyses. Figure 2 shows the corresponding PLS score plot of the two first principal components that are plotted against each other for all samples measured in the second study. In the score plot each point represents one 1 sample. Based on the list of 102 protein biomarkers a good separation between sensitizer and irritant treated samples was achieved. It can be seen that that samples treated with Nickel had the greatest relative distance from the centre of the plot, whereas TMTD showed a smaller distance to the centre of the plot indicating that in most samples TMTD elicited samples a smaller response.

### Example 3 - Targeted biomarker measurements

As an alternative approach to find predictive markers for sensitizing potential we measured the concentration of biomarkers using commercially available immunoassays to measure the levels of three biomarkers. The three proteins to be measured by Western Blot or ELISA were selected on the basis of a review of literature references to biomarkers of stress toxicity pathways. Cells were cultured as described in Example 1 and the cell extract was used for direct analysis by ELISA or Western Blot. All kits were used in accordance with manufacturer's instructions.

### Heat shock 70 kDa protein 1 (HSP70, HSPA1A; HSPA1B)

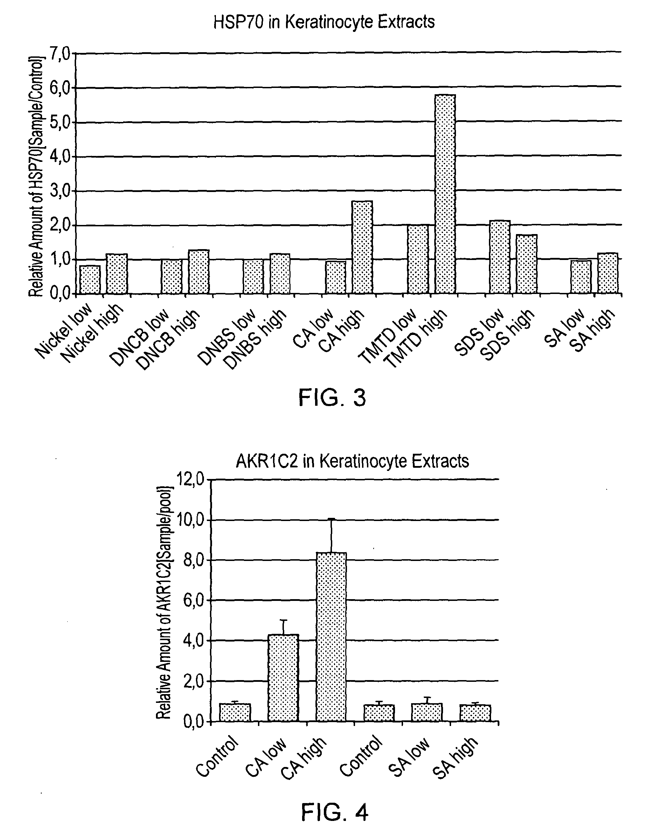

Heat shock proteins (HSPs) function as molecular chaperones by preventing misfolding and aggregation of proteins essential and by assisting proper folding of proteins. The expression of HSPs is increased by heat, toxins and oxidative stress. The 70 kilodalton heat shock proteins (Hsp70s) comprise three members HSPA1A, HSPA1B, and HSPA1L. HSP70 is expressed in the skin and inhibition of HSP70 in mice using HSP70 specific antibodies resulted in a reduction in the 1-fluoro-2,4-dinitrobenzene contact hypersensitivity response (Yusuf et al. 2009). Effects of contact sensitizers and irritants on HSP70 production were determined using an HSP70-specific ELISA (Assay Designs Inc. Ann Arbor, USA). Based on the general stress response pathway implicated in the induction of HSP70 expression, it might be expected to be a marker for all classes of chemical allergens. Surprisingly, the results show that HSP70 is most strongly induced by TMTD and CA, whereas the response to DNCB, Nickel and DNCB was small, only (Figure 3).

### Metallothionein-1G (MT1G)

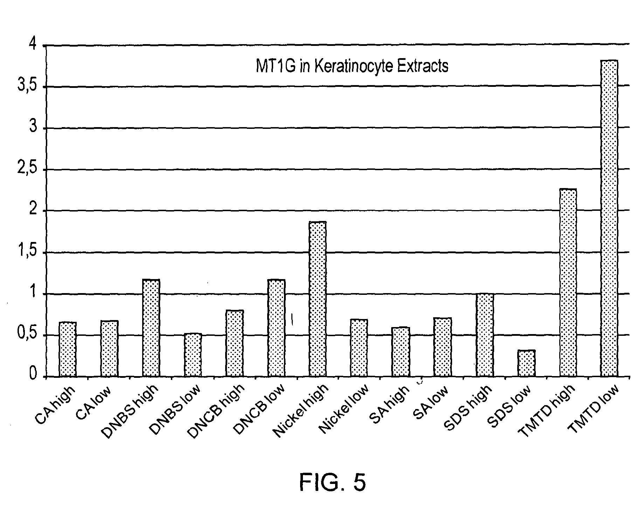

Metallothioneins (MTs) are low molecular weight, cysteine-rich proteins that sequester metal including Zinc and Nickel and protect cells from metal toxicity. MT expression is induced by metal ions and in oxidative stress models. Furthermore, MT appears to play cytoprotective role in mouse model for antigen-related airway inflammation (reviewed in Inoue et al. 2009). Effects of contact sensitizers and irritants on MT1G production was determined by Western Blot analysis using MT1G specific antibodies (clone UC1MT, Biozol, Eching, Germany). Surprisingly, the results showed that MT1G was most strongly induced in keratinocytes exposed to TMTD compared to the metal allergen Nickel and DNCB (Figure 5).

### Aldo-keto reductase C1 (AKR1C2)

Aldo-keto reductase family 1, member C2 (AKR1C2) is a member of the aldo/keto reductase superfamily, whose members catalyze the conversion of aldehydes and ketones to their corresponding alcohols using NADH and/or NADPH as cofactors. Upregulation of AKR1C2 gene expression has been described in dendritic cells exposed to skin sensitizers including DNBS and Nickel (Gildea et al. 2006). Effects of contact sensitizers and irritants on AKR1C2 production was determined by Western Blot analysis using AKR1C2-specific rabbit polyclonal antibodies (Antibodies-Online, Aachen, Germany). In contrast, to dendritic cells AKR1C3 was only induced in CA exposed samples (Figure 4). These results indicate that substantial differences exist in the induction of response pathway to chemical sensitizers in dendritic cells and keratinocytes.

### Example 5 - Biomarker Panel for General Sensitizers

The results of Examples 1 - 4 have identified a panel of 102 proteins (Table 1, Groups 1-3) for the discrimination of chemical sensitizers from irritant or control chemicals. The method described to determine the panel of 102 biomarkers using reference chemicals can now be used in a revised form to test new or previously untested chemical agents to determine their potential as allergens, sensitizers or non-sensitizing. According to the present invention the method may employ measuring the concentration of biomarkers chosen from table 1 in test sets of samples exposed to new chemicals or new chemicals in combination with reference compounds as positive and negative controls. Typically, when evaluating a new test chemical the analysis should be performed using a combination of biomarkers from Table 1, especially selecting biomarkers from group 1 or group 2. Use of a panel of biomarkers selected from group 1 and group 2 will ensure the most robust discrimination between sensitizer, irritant and control. When the selected biomarkers perform well on a new chemical compound one would retain the combination of biomarkers.
Alternatively it is possible test other combinations of biomarkers from group 1 or 2 in an iterative process. It is also possible to reject biomarkers from group 1 or 2 and include biomarkers from group 3. This iterative process will continue until a good classification model is produced.
In certain instances higher doses of chemical sensitizers such as DNCB can as well cause skin irritation. It is thought that chemical irritants such as SDS cause skin inflammation through activation of the innate immune response.

When determining the sensitizing potential of a chemical the analysis may also involve testing of increasing exposure concentrations including irritating and non-irritating concentrations.

It is also possible to measure the irritating potential of chemical by measuring the concentration of biomarkers detailed in Table 1. Typically, the analysis should be performed using biomarkers linked to inflammatory and cellular stress processes. In particular, biomarkers selected from group 2 that are induced following exposure to SDS may be useful. This may involve but is not limited to measuring the concentration of SerpinB2 (plasminogen activator inhibitor-2, PAI-2), a serine protease inhibitor involved in cutaneous wound repair, EPS8 (epidermal growth factor receptor kinase substrate 8), a protein involved in actin remodelling and CTSB (Cystatin B), an intracellular inhibitor of cystein proteases. Of particular value to the present invention is ANXA1 (Annexin 1) a plasma membrane associated protein involved inflammatory processes. Other markers for irritants include VDAC3 (Voltage-dependent anion-selective channel protein 3), a pore-forming proteins mainly located in the mitochondrial outer membrane, and RPL14 (60S ribosomal protein L14). The inclusion and combination of proteins modulated by sensitizing or irritating chemicals will allow to correctly predict the sensitizing or irritating potential of new chemicals.

### Example 6 - Biomarker Panel for Contact Sensitizers

Within the panel of general markers of sensitizing potential it is also possible to select the strongest discriminant markers correlating with contact sensitizer effect. Using PLS- DA a sub-group of 12 proteins providing the strongest separation of skin sensitizers from all other classes was identified (Table 1, Group 1). It was thus possible to perform a targeted analysis to measure just these 12 proteins to detect known skin sensitizers and demonstrate whether an unknown test chemical or combination of chemicals possesses skin sensitizing potential.

### Example 7 - Selective Reaction Monitoring (SRM) assays for the multiplexed analysis of biomarkers

To approach the complex and variable cellular response to different chemical sensitizers, assays are required that allow simultaneous analysis of several biomarkers representing different cellular response pathways. SRM-based approaches are an attractive alternative to ELISAs due to the sensitivity and selectivity of the technique, the capacity to multiplex and the limited availability of antibodies. Here, signature peptides unique to the protein of interest are measured to provide quantitative information of that protein in the sample. Changes in peptide abundance in response to chemical exposure experiments can be determined using typical isotopic TMT-SRM workflows. Here, quantitation is based on the relative MS intensities of the sample peptide labelled with TMTzero versus an internal reference sample labelled with TMTsixplex heavy isotope. Four proteins were selected to develop multiplexed SRM assays: Thymosin beta 10 (TYB10), Protein S100-A8 (S100A8), Protein S100-A9 (S100A9) and Heat shock cognate 71 kDa protein (HSPA8).

### Methods

### Selection of candidate peptides for SRM quantitation

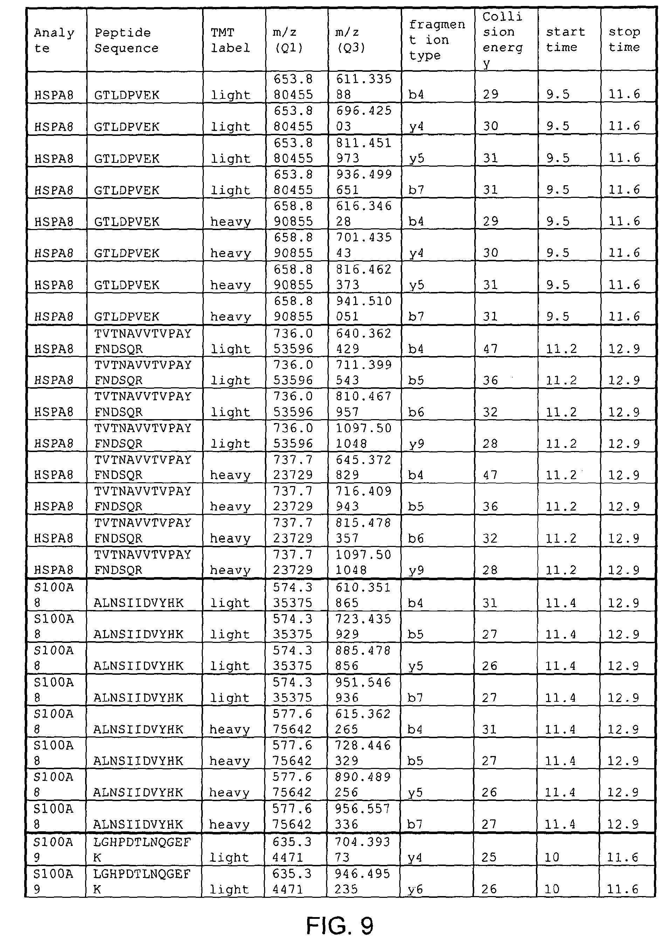

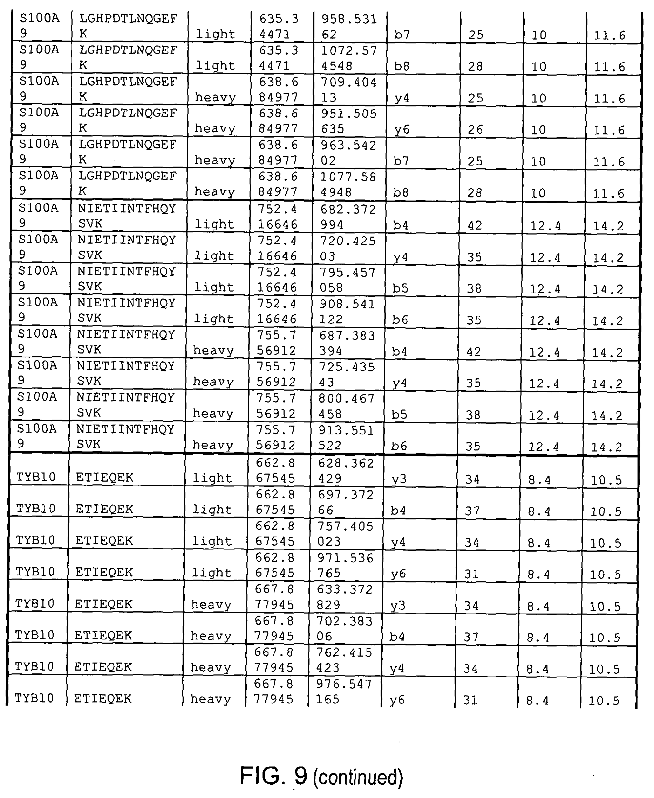

Using existing MS/MS data, most frequently observed specific peptides were selected for quantitation. If possible at least three peptides per protein were selected for SRM development. The representative peptides for the four selected proteins (HSPA8, S100A8, S100A9 and TYB10) are shown in Table 5 (Figure 9). Criteria for selection included; no missed cleavages with trypsin and no variable modifications (in-vivo or experimental).

### Preparation of samples

Keratinocyte samples are treated with sensitizer and irritant or are left untreated as in the discovery phase. A pool sample is digested with trypsin and labelled with TMTsixplex to produce the heavy-labelled version of peptides to act as a reference for quantitation. Test samples are digested and labelled with TMTzero to produce the light labelled version of peptides. 15 µg each of the pool and test sample are afterwards mixed and undergo subsequent purification by solid- phase extraction and strong cation exchange using volatile buffers.

### SRM analysis of samples

The mixed heavy and light labelled samples are resuspended in 5% Acetonitril (=ACN), 0.2% Formic acid (=FA)and infused into an Accela 1250 Liquid Chromatography (LC) system coupled to a TSQ Vantage triple stage quadrupole mass spectrometer (Thermo Fisher) and SRM data are acquired. Corresponding TMTsixplex-labelled and TMTzero-labelled fragment ion masses are calculated and MS instrument parameters optimised for individual Q1 and Q3 transition pairs. A pooled cell lysate sample is digested, labelled with TMTsixplex and combined with the TMTzero-labelled test sample. Using accurate retention times for each peptide, the SRM cycle time is 1.5 seconds with retention time windows used to maximise the scan time given to each SRM transition. Including washes and time to equalibrate the column, the total run time of the method is 23 minutes. Declustering voltage is set to 5 Volt, Peak width (FWHM) is set to 0.5 and Chrome filter Peak width is set to 6 seconds. Specific parameters for the SRM transitions are listed in Table 5 (Figure 9).

### Data analysis

SRMs are visualised through Skyline version 1.2.0.3425 (https://skyline.gs.washington.edu/labkey/project/home/softwar e/Skyline/begin.view) and all peak matching visually verified. Integrated peak areas are exported into Microsoft Excel. Transitions are summed to give a total intensity for all transitions for each peptide. The amount of endogenous (light) peptide is calculated based on the peak area ratio relative to the internal heavy-labelled reference sample.

### References

Corsini E, Mitjans M, Galbiati V, Lucchi L, Galli CL, Marinovich M (2009). Use of IL-18 production in a human keratinocyte cell line to discriminate contact sensitizers from irritants and low molecular weight respiratory allergens. Toxicol In Vitro. 23(5):789-96.
Emter R, Ellis G, Natsch A (2010). Performance of a novel keratinocyte-based reporter cell line to screen skin sensitizers in vitro. Toxicol Appl Pharmacol. 245(3):281-90.
Gildea LA, Ryan CA, Foertsch LM, Kennedy JM, Dearman RJ, Kimber I, Gerberick GF. (2006) Identification of gene expression changes induced by chemical allergens in dendritic cells: opportunities for skin sensitization testing. J Invest Dermatol. 126(8):1813-22.
Inoue K, Takano H, Shimada A, Satoh M (2009). Metallothionein as an anti-inflammatory mediator. Mediators Inflamm. 2009:101659.
Kimber, I., Hilton, J., Dearman, R.J., Gerberick, G.F., Ryan,C.A., Basketter, D.A., Scholes, E.W., Ladies, G.S., Loveless, S.E., House, R.V., Guy, A., (1995). An international evaluation of the murine local lymph node assay and comparison of modified procedures. Toxicology 103, 63-73.
Magnusson, B., Kligman, A.M., (1969). The identification of contact allergens by animal assay. The guinea pig maximization test. J. Invest. Dermatol. 52, 268-276.
Natsch A, Emter R (2008). Skin sensitizers induce antioxidant response element dependent genes: application to the in vitro testing of the sensitization potential of chemicals. Toxicol Sci. 102(1):110-9.
Natsch A. (2010). The Nrf2-Keap1-ARE toxicity pathway as a cellular sensor for skin sensitizers--functional relevance and a hypothesis on innate reactions to skin sensitizers. Toxicol Sci. 113:284-92.
Vandebriel RJ, Pennings JL, Baken KA, Pronk TE, Boorsma A, Gottschalk R, Van Loveren H (2010). Keratinocyte gene expression profiles discriminate sensitizing and irritating compounds. Toxicol Sci. 2010 117(1):81-9.
Van Och FM, Van Loveren H, Van Wolfswinkel JC, Machielsen AJ, Vandebriel RJ (2005).Assessment of potency of allergenic activity of low molecular weight compounds based on IL-1alpha and IL-18 production by a murine and human keratinocyte cell line. Toxicology. 210(2-3):95-109.
Yoshikawa Y, Sasahara Y, Kitano Y, Kanazawa N, Shima H, Hashimoto-Tamaoki T (2010). Upregulation of genes orchestrating keratinocyte differentiation, including the novel marker gene ID2, by contact sensitizers in human bulge- derived keratinocytes. J Biochem Mol Toxicol. 24(1):10-20.
Yusuf N, Nasti TH, Huang CM, Huber BS, Jaleel T, Lin HY, Xu H, Elmets CA (2009). Heat shock proteins HSP27 and HSP70 are present in the skin and are important mediators of allergic contact hypersensitivity. J Immunol. 182(1):675-83.
Kimber I, Basketter DA, McFadden JP, Dearman RJ (2011). Characterization of skin sensitizing chemicals: a lesson learnt from nickel allergy. J Immunotoxicol. (1):1-2.
Bauch C, Kolle SN, Fabian E, PAchel C. Ramirez T, Wiench B, Wruck CJ, van Ravenzwaay B, Landsiedel R (2011). Intralaboratory validation of four in vitro assay for the production of the skin sensitizing potential of chemicals. Toxicology in vitro. 25:1162-1168.
Ellis G, Emter R, Natsch A (2009). Development of a high-throughput keratinocyte-based standard assay to detect on ARE-dependent gene activity. Toxicology Letters S69.
Helm S, Pankert P, Schnoelzer M, Thierse H-J (2007). Proteomic approach to characterize the effect of skin sensitizers on human primary keratinocytes. Toxicology Letters S8.
Lambrechts N, Vanheel H, Nelissen I, Witters H, Van Den Heuvel R, Van Tendeloo V, Schoeters G, Hooyberghs J (2010). Assessment of Chemical Skin-Sensitizing Potency by an in vitro assay based on human dendritic cells. Toxicological Sciences 116(1):122-129.
Lambrechts N, Verstraelen S, Lodewyckx H, Felicio A, Hooyberghs J, Witters H, Van Tendeloo V, Van Cauwenberge P, Nelissen I, Van Den Heuvel R, Schoeters G (2009). THP-1 monocytes but not macrophages as a potential alternative for CD34+ dendritic cells to identify chemical skin sensitizers. Toxicology and applied Pharmacology 236:221-230.

## Claims

1. An in vitro method for determining the sensitizing potential of a test compound, comprising the steps of
(a) contacting said test compound with a cell;
(b) determining a change in the level of expression of glyceraldehyde-3-phosphate dehydrogenase (GAPDH) and further marker proteins; and
(c) determining the sensitizing potential of said test compound based on said change in level of expression wherein an increase in the level of expression of said GAPDH and a change in the level of said further marker proteins is indicative of said test compound having sensitizing potential,
wherein said further marker proteins are selected from Table 1, Table 1 (A) Group 1; Table 1 (B) Group 2; or Table 1 (C) Group 3 or a combination thereof, and wherein the cell is representative of a mammalian skin cell.

2. A method according to claim 1 wherein the said mammalian skin cell is selected from the group consisting of a primary keratinocyte, a keratinocyte derived cell line, an epidermal keratinocyte, bulge-derived keratinocytes, foreskin keratinocytes, a human cell having keratinocyte properties, a cell derived from HaCaT cell line and a cell derived from NCTC2544 cell line.

3. A method according to claim 1 or claim 2 wherein said further marker proteins are selected from Table 1 (A) Group 1 or are selected from Table 1 (B) Group 2.

4. A method according to any one of the preceding claims wherein step (b) includes comparing the level of expression of GAPDH and further markers proteins with a reference level.

5. A method according to any one of the preceding claims wherein step (b) includes contacting the cell with at least one specific binding member that selectively binds to said marker protein or nucleic acid sequence encoding said marker protein; and detecting and/or quantifying a complex formed by said specific binding member and the marker protein or nucleic acid sequence encoding said marker protein.

6. A method according to any one of the preceding claims wherein said determination step includes preparing a standard curve using standards of known expression levels of the GADPH and the further marker proteins and comparing the reading obtained with the cell contacted with the test compound so as to derive a measure of the change in level of expression of GAPDH and the further marker proteins.

7. A method according to claim 5 or claim 6 wherein said specific binding member may be an antibody or fragment thereof that specifically and selectively binds to said marker protein.

8. A method according to claim 7 wherein said specific binding member is an auto-antibody or fragment thereof that specifically and selectively binds to GADPH wherein said auto- antibody is prepared from a blood sample obtained from a patient with skin irritation or allergy.

9. A method according to claim 5 or claim 6 wherein the specific binding member is an aptamer.

10. A method according to any one of claims 1 to 4 wherein step (b) is performed by mass spectrometry.

11. A method according to any one of claim 1 to 4 wherein step (b) is performed by Selected Reaction Monitoring using one or more transitions for protein marker derived peptides;
(i) comparing the peptide levels in the cell under test with peptide levels previously determined to represent sensitivity of the cell, and
(ii) determining the sensitizing potential of the test compound based on changes in expression of GAPDH and said further marker proteins; wherein an increase in expression of GAPDH is indicative of said test compound having sensitizing potential.

12. A method according to claim 11 wherein step (i) includes determining the amount of marker protein derived peptides from the cell under test with known amounts of corresponding synthetic peptides, wherein the synthetic peptides are identical in sequence to the peptides obtained from the cell except for a label.

13. A method according to claim 12 wherein the label is a tag of a different mass or a heavy isotope.

14. A method according to any one of claims 11 to 13, wherein the one or more transitions for the protein marker derived peptides comprise one or more transitions from Table 5.

15. A method according to any one of claims 1 to 6 wherein step (b) comprises determining the presence or amount of mRNA encoding GAPDH and said further marker proteins in the cell following contact with the test compound.

16. A method according to claim 15 wherein the presence or amount of mRNA is determined using a primer or probe which selectively binds to the sequence of the protein marker encoding gene or complement thereof.

17. A method according to any one of claims 5 to 10 and 15 to 16 wherein the binding member is immobilised on a solid support.

18. A kit for use in determining the sensitizing potential of a test compound in vitro, said kit allowing the user to determine the level of expression of GAPDH and further marker proteins or fragments thereof provided in Table 1, in a cell under test; the kit comprising
(a) a set of reference peptides in an assay compatible format wherein each peptide in the set is uniquely representative of each of GAPDH and the further marker proteins provided in Table 1, Table 1 (A) Group 1; Table 1 (B) Group 2; or Table 1 (c) Group 3 or a combination thereof; and, optionally
(b) one or more components selected from the group consisting of washing solutions, diluents and buffers.

19. A method according to claim 1 for the diagnosis or prognostic monitoring of contact sensitizing potential or irritating potential by an allergen or irritant on an individual exposed to said allergen or irritant; wherein the cell is obtained from said individual.

## Patentansprüche

1. in-vitro-Verfahren zum Ermitteln des Sensibilisierungspotentials einer Testverbindung, das die folgenden Schritte beinhaltet:
(a) Inkontaktbringen der genannten Testverbindung mit einer Zelle;
(b) Ermitteln einer Änderung des Expressionsniveaus von Glyceraldehyd-3-phosphat-dehydrogenase (GAPDH) und weiterer Marker-Proteine; und
(c) Ermitteln des Sensibilisierungspotentials der genannten Testverbindung auf der Basis der genannten Änderung des Expressionsniveaus, wobei eine Zunahme des Expressionsniveaus der genannten GAPDH und eine Änderung des Niveaus der genannten weiteren Marker-Proteine anzeigt, dass die genannte Testverbindung Sensibilisierungspotential hat,
wobei die genannten weiteren Marker-Proteine aus Tabelle 1, Tabelle 1 (A) Gruppe 1; Tabelle 1 (B) Gruppe 2 oder Tabelle 1 (C) Gruppe 3 oder einer Kombination davon ausgewählt sind und wobei die Zelle für eine Säugetierhautzelle repräsentativ ist.

2. Verfahren nach Anspruch 1, wobei die genannte Säugetierhautzelle ausgewählt ist aus der Gruppe bestehend aus einem primären Keratinozyt, einer Keratinozytabgeleiteten Zelllinie, einem epidermalen Keratinozyt, von wulstabgeleiteten Keratinozyten, Vorhautkeratinozyten, einer menschlichen Zelle mit Keratinozyteneigenschaften, einer von der HaCaT-Zelllinie abgeleiteten Zelle und einer von der NCTC25A.4-Zelllinie abgeleiteten Zelle.

3. Verfahren nach Anspruch 1 oder Anspruch 2, wobei die genannten weiteren Marker-Proteine aus Tabelle 1 (A) Gruppe 1 oder aus Tabelle 1 (B) Gruppe 2 ausgewählt sind.

4. Verfahren nach einem der vorherigen Ansprüche, wobei Schritt (b) das Vergleichen des Expressionsniveaus von GAPDH und weiterer Marker-Proteine mit einem Referenzniveau beinhaltet.

5. Verfahren nach einem der vorherigen Ansprüche, wobei Schritt (b) das Inkontaktbringen der Zelle mit wenigstens einem spezifischen Bindungselement beinhaltet, das sich selektiv an das genannte Marker-Protein oder an die das genannte Marker-Protein codierende Nukleinsäuresequenz bindet; und Nachweisen und/oder Quantifizieren eines Komplexes, der von dem genannten spezifischen Bindungselement und dem Marker-Protein oder der das genannte Marker-Protein codierenden Nukleinsäuresequenz gebildet wird.

6. Verfahren nach einem der vorherigen Ansprüche, wobei der genannte Ermittlungsschritt das Erzeugen einer Standardkurve mit Standards von bekannten Expressionsniveaus der GAPDH und der weiteren Marker-Proteine und das Vergleichen des Messwerts, der mit der Zelle erhalten wurde, die mit der Testverbindung in Kontakt gebracht wurde, beinhaltet, um ein Maß für die Änderung des Expressionsniveaus von GAPDH und der weiteren Marker-Proteine abzuleiten.

7. Verfahren nach Anspruch 5 oder Anspruch 6, wobei das genannte spezifische Bindungselement ein Antikörper oder ein Fragment davon sein kann, der/das sich spezifisch und selektiv an das genannte Marker-Protein bindet.

8. Verfahren nach Anspruch 7, wobei das genannte spezifische Bindungselement ein Autoantikörper oder ein Fragment davon ist, der/das sich spezifisch und selektiv an GAPDH bindet, wobei der genannte Autoantikörper von einer von einem Patienten mit Hautreizung oder-allergie erhaltenen Blutprobe hergestellt wird.

9. Verfahren nach Anspruch 5 oder Anspruch 6, wobei das spezifische Bindungselement ein Aptamer ist.

10. Verfahren nach einem der Ansprüche 1 bis 4, wobei Schritt (b) durch Massenspektrometrie ausgeführt wird.

11. Verfahren nach einem der Ansprüche 1 bis 4, wobei Schritt (b) durch selektierte Reaktionsüberwachung (SRM) mittels eines oder mehrerer Übergänge für Protein-Marker-abgeleitete Peptide ausgeführt wird;
(i) Vergleichen der Peptidniveaus in der getesteten Zelle mit Peptidniveaus, von denen zuvor festgestellt wurde, dass sie Empfindlichkeit der Zelle repräsentieren, und
(ii) Ermitteln des Sensibilisierungspotentials der Testverbindung auf der Basis von Änderungen der Expression von GAPDH und der genannten weiteren Marker-Proteine; wobei eine Zunahme der GAPDH-Expression anzeigt, dass die genannte Testverbindung Sensibilisierungspotential hat.

12. Verfahren nach Anspruch 11, wobei Schritt (i) das Ermitteln der Menge an Marker-Protein-abgeleiteten Peptiden von der getesteten Zelle mittels bekannter Mengen an entsprechenden synthetischen Peptiden beinhaltet, wobei die synthetischen Peptide in der Sequenz mit den von der Zelle gewonnenen Peptiden identisch sind, mit Ausnahme einer Kennung.

13. Verfahren nach Anspruch 12, wobei die Kennung ein Tag einer anderen Masse oder ein schweres Isotop ist.

14. Verfahren nach einem der Ansprüche 11 bis 13, wobei die ein oder mehreren Übergänge für die Protein-Marker-abgeleiteten Peptide einen oder mehrere Übergänge aus Tabelle 5 umfassen.

15. Verfahren nach einem der Ansprüche 1 bis 6, wobei Schritt (b) das Ermitteln der Anwesenheit oder Menge von GAPDH-codierender mRNA und der genannten weiteren Marker-Proteine in der Zelle nach einem Kontakt mit der Testverbindung beinhaltet.

16. Verfahren nach Anspruch 15, wobei die Anwesenheit oder Menge an mRNA mit einem Primer oder einer Sonde ermittelt wird, der/die sich selektiv an die Sequenz des den Protein-Marker codierenden Gens oder eines Komplements davon bindet.

17. Verfahren nach einem der Ansprüche 5 bis 10 und 15 bis 16, wobei das Bindungselement auf einem festen Träger immobilisiert wird.

18. Kit zur Verwendung beim Ermitteln des Sensibilisierungspotentials einer Testverbindung in vitro, wobei es der genannte Kit dem Benutzer gestattet, das Expressionsniveau von GAPDH und weiterer Marker-Proteine oder Fragmente davon gemäß Tabelle 1 in einer Zelle unter Test zu ermitteln; wobei der Kit Folgendes umfasst:
(a) einen Satz von Referenzpeptiden in einem Assay-kompatiblen Format, wobei jedes Peptid in dem Satz eindeutig für jedes aus GAPDH und den weiteren Marker-Proteinen gemäß Tabelle 1, Tabelle 1 (A) Gruppe 1; Tabelle 1 (B) Gruppe 2; oder Tabelle 1 (C) Gruppe 3 oder einer Kombination davon repräsentativ ist; und fakultativ
(b) eine oder mehrere Komponenten, ausgewählt aus der Gruppe bestehend aus Waschlösungen, Verdünnungsmitteln und Puffern.

19. Verfahren nach Anspruch 1 zur Diagnose oder prognostischen überwachung des Kontaktsensibilisierungs- oder Reizpotentials durch ein Allergen oder einen Reizstoff bei einem Individuum, das dem genannten Allergen oder Reizstoff ausgesetzt ist; wobei die Zelle von dem genannten Individuum gewonnen wird.

## Revendications

1. Procédé in vitro de détermination du potentiel de sensibilisation d'un composé de test, comprenant les étapes consistant à
(a) mettre en contact ledit composé de test avec une cellule ;
(b) déterminer un changement dans le niveau d'expression de la glycéraldéhyde-3-phosphate déshydrogénase (GAPDH) et d'autres protéines de marquage ; et
(c) déterminer le potentiel de sensibilisation dudit composé de test sur la base dudit changement dans le niveau d'expression, une augmentation du niveau d'expression de ladite GAPDH et un changement dans le niveau desdites autres protéines de marquage étant indicatifs dudit composé de test ayant le potentiel de sensibilisation,
lesdites autres protéines de marquage étant choisies dans le Tableau 1, le Tableau 1(A) Groupe 1 ; Tableau 1(B) Groupe 2 ; ou Tableau 1 (C) Groupe 3 ou une combinaison de ceux-ci, et la cellule étant représentative d'une cellule cutanée de mammifère.

2. Procédé selon la revendication 1, dans lequel ladite cellule cutanée de mammifère est choisie dans le groupe comportant un kératinocyte primaire, une lignée cellulaire dérivée de kératinocytes, un kératinocyte épidermique, des kératinocytes dérivés du ventre, kératinocytes de prépuce, une cellule humaine ayant des propriétés de kératinocytes, une cellule dérivée de la lignée cellulaire HaCaT, et une cellule dérivée de la lignée cellulaire NCTC2544.

3. Procédé selon la revendication 1 ou la revendication 2, dans lequel lesdites autres protéines de marquage sont choisies dans le Tableau 1(A) Groupe 1 ou sont choisies dans le Tableau 1(B) Groupe 2.

4. Procédé selon l'une quelconque des revendications précédentes, dans lequel l'étape (b) comprend la comparaison du niveau d'expression de GAPDH, et d'autres protéines de marquage avec un niveau de référence.

5. Procédé selon l'une quelconque des revendications précédentes, dans lequel l'étape (b) comprend la mise en contact de la cellule avec au moins un élément de liaison spécifique qui se lie sélectivement à ladite protéine de marquage ou à ladite la séquence d'acide nucléique codant ladite protéine de marquage ; et la détection et/ou la quantification d'un complexe formé par ledit élément de liaison spécifique et la protéine de marquage ou la séquence d'acide nucléique codant lesdites protéines de marquage.

6. Procédé selon l'une quelconque des revendications précédentes, dans lequel ladite étape de détermination comprend la préparation d'une courbe étalon en utilisant des étalons de niveaux d'expression connus de la GADPH et des autres protéines de marquage et la comparaison de la valeur obtenue avec la cellule en contact avec le composé de test de manière à obtenir une mesure du changement dans le niveau d'expression de GAPDH et des autres protéines de marquage.

7. Procédé selon la revendication 5 ou la revendication 6, dans lequel ledit membre de liaison spécifique peut être un anticorps ou un fragment de celui-ci qui se lie spécifiquement et sélectivement à ladite protéine de marquage.

8. Procédé selon la revendication 7, dans lequel ledit élément de liaison spécifique est un auto-anticorps ou un fragment de celui-ci qui se lie spécifiquement et sélectivement à GADPH et dans lequel ledit auto-anticorps est préparé à partir d'un échantillon de sang prélevé chez un patient présentant une irritation ou allergie de la peau.

9. Procédé selon la revendication 5 ou la revendication 6, dans lequel l'élément de liaison spécifique est un aptamère.

10. Procédé selon l'une quelconque des revendications 1 à 4, dans lequel l'étape (b) est effectuée par spectrométrie de masse.

11. Procédé selon l'une quelconque des revendications 1 à 4, dans lequel l'étape (b) est effectuée par la méthode SRM (Selected Reaction Monitoring) à l'aide d'une ou plusieurs transitions de peptides dérivés de la protéine de marquage ;
(i) comparaison des niveaux de peptide dans la cellule en cours de test avec les niveaux de peptides préalablement déterminés pour représenter la sensibilité de la cellule, et
(ii) détermination du potentiel de sensibilisation du composé de test sur la base de changements dans l'expression de GAPDH et desdites autres protéines de marquage ; une augmentation de l'expression de GAPDH étant indicative dudit composé de test ayant le potentiel de sensibilisation.

12. Procédé selon la revendication 11, dans lequel l'étape (i) comprend la détermination de la quantité de peptides dérivés de la protéine de marquage provenant de la cellule en cours de test avec des quantités connues de peptides synthétiques correspondants, les peptides synthétiques étant identiques en séquence aux peptides obtenus à partir de la cellule sauf pour un marqueur.

13. Procédé selon la revendication 12, dans lequel le marqueur est un marqueur d'une masse différente ou d'un isotope lourd.

14. Procédé selon l'une quelconque des revendications 11 à 13, dans lequel les une ou plusieurs transitions pour les peptides dérivés de la protéine de marquage comprennent une ou plusieurs transitions du Tableau 5.

15. Procédé selon l'une quelconque des revendications 1 à 6, dans lequel l'étape (b) comprend la détermination de la présence ou de la quantité d'ARNm codant la GAPDH et lesdites autres protéines de marquage dans la cellule suivant le contact avec le composé de test.

16. Procédé selon la revendication 15, dans lequel la présence ou la quantité d'ARNm est déterminée à l'aide d'une amorce ou d'une sonde qui se lie sélectivement à la séquence du gène codant la protéine de marquage ou un complément de celle-ci.

17. Procédé selon l'une quelconque des revendications 5 à 10 et 15 à 16, dans lequel l'élément de liaison est immobilisé sur un support solide.

18. Kit destiné à être utilisé dans la détermination du potentiel de sensibilisation d'un composé de test in vitro, ledit kit permettant à l'utilisateur de déterminer le niveau d'expression de GAPDH, et d'autres protéines de marquage ou de leurs fragments fournis dans le Tableau 1, dans une cellule en cours de test ; le kit comprenant
(a) un ensemble de peptides de référence dans un format compatible avec l'essai, chaque peptide dans l'ensemble étant représentatif de façon unique de chacun parmi GAPDH et les autres protéines de marquage fournis dans le Tableau 1, le Tableau 1(A) Groupe 1 ; le Tableau 1(B) Groupe 2 ; ou le Tableau 1(c) Groupe 3 ou une combinaison de ceux-ci ; et, facultativement
(b) un ou plusieurs composants choisis dans le groupe comportant des solutions de lavage, des diluants et des tampons.

19. Procédé selon la revendication 1 servant à surveiller, en vue d'un diagnostic ou d'un pronostic, le potentiel de sensibilisation ou d'irritation par contact par un allergène ou un irritant sur un individu exposé audit allergène ou irritant ; la cellule étant prélevé chez ledit individu.
